Europäisches Patentamt
European Patent Office
Office européen des brevets

(19)

(11) Publication number : **0 495 674 A2**

(12) **EUROPEAN PATENT APPLICATION**

(21) Application number : **92300429.5**

(22) Date of filing : **17.01.92**

(51) Int. Cl.$^5$ : **C12N 15/12**, C12P 21/02, C07K 13/00, C12Q 1/68

(30) Priority : **18.01.91 US 642991**
**10.01.92 US 816270**

(43) Date of publication of application :
**22.07.92 Bulletin 92/30**

(84) Designated Contracting States :
**AT BE CH DE DK ES FR GB GR IT LI LU NL PT SE**

(71) Applicant : **Bristol-Myers Squibb Company**
**345 Park Avenue**
**New York, N.Y. 10154 (US)**

(72) Inventor : **Purchio, Anthony F.**
**801 33rd Avenue East**
**Seattle, Washington 98112 (US)**
Inventor : **Brunner, Amy M.**
**4345 32nd Avenue West No. 305**
**Seattle, Washington 98199 (US)**
Inventor : **Chinn, Joyce**
**3107 25th Avenue South**
**Seattle, Washington 98144 (US)**
Inventor : **Neubauer, Michael G.**
**558 Highland Drive**
**Seattle, Washington 98109 (US)**

(74) Representative : **Jones, Alan John et al**
**CARPMAELS & RANSFORD 43 Bloomsbury Square**
**London, WC1A 2RA (GB)**

(54) **TGF-beta induced gene family.**

(57) A new gene family induced by TGF-beta is disclosed. Two new genes, designated βIG-M1 and βIG-M2, are induced in response to TGF-β1 treatment of mouse embryo fibroblasts. These genes encode proteins containing about 345 to about 380 amino acid residues, with a molecular weight of about 37,000 to about 48,000 daltons and about 38 cysteine residues. The induced proteins share about 50% homology with each other and significant homology with a v-src induced protein in chicken embryo fibroblasts designated CEF-10. These proteins may be involved in producing some of the growth and differention modulating effects of TGF-β1.

```
βIG-M1      CIVQTTSWSQCSKSCGTGISTRVT-------NDNPECRL-VKETRICEVR    42
CEF12CS     CIVQTTSWSQCSKTCGTGISTRVT-------NDNPDCKL-IKETRICEVR    42
βIG-M2      CLVQTTEWSACSKTCGMGISTRVT-------NDNTFCRL-EKQSRLCMVR    42
PFALCIPACS  NSI-STEWSPCSVTCGNGIQVRIKPGSANKPKDELDYEN-DIEKKICKME    48
PROPERDCSR  WSX-WSPWSPCSVTCSXGXQXXXRXRXCXXPAPXX-GXPCAGXAXXXXXQ    48
THROMBOCS   WSH-WSPWSSCSVTCGDGV--ITRIRLCNSPSPQMNGKPC--ECEARETK    45
PFALTRAPCS  CGV-WDEWSPCSVTCGKGTRSRKREILHEG---------CTSEIQEQ---    37
C7COMPCS    WDF-YAPWSECN-GCTKTQTRRRSVAVYG----QYGGQPCVG--NAFETQ    42
                  . **  *. .*. .
                   region II of CS protein

βIG-M1      PCGQPVYSSLKKGKKCSK    60
CEF12CS     PCGQPSYASLKKGKKCTK    60
βIG-M2      PCEADLEENIKKGKKCIR    60
PFALCIPACS  KCSSVF-----------N    55
PROPERDCSR  ACXXXXPCPXX-G-----    60
THROMBOCS   ACKKDA-CPIN-G-----    56
PFALTRAPCS  -CE-EERCPPKWE-----    48
C7COMPCS    SCEPTRGCPTEEG--C--    56
             *
```

FIGURE 7

EP 0 495 674 A2

Jouve, 18, rue Saint-Denis, 75001 PARIS

## TECHNICAL FIELD OF THE INVENTION

The present invention is directed to the induction of a new gene family in response to TGF-beta administration to target cells in culture. Two specifically induced genes were isolated and characterized.

## BACKGROUND OF THE INVENTION

Transforming growth factor-β1 (TGF-β1) is a multifunctional regulator of cell growth and differentiation. It is capable of causing diverse effects such as inhibition of the growth of monkey kidney cells, (Tucker, R.F., G.D. Shipley, H.L. Moses & R.W. Holley (1984) Science 226:705-707) inhibition of growth of several human cancer cell lines, (Roberts, A.B., M.A. Anzano, L.M. Wakefiled, N.S. Roches, D.F. Stem & M.B. Sporn (1985) Proc. Natl. Acad. Sci. USA 82:119-123; Ranchalis, J.E., L.E. Gentry, Y. Agawa, S.M. Seyedin, J. McPherson, A. Purchio & D.R. Twardzik (1987) Biochem. Biophys. Res. Commun. 148:783-789) inhibition of mouse keratinocytes, (Coffey, R.J., N.J. Sipes, C.C. Bascum, R. Gravesdeal, C. Pennington, B.E. Weissman & H.L. Moses (1988) Cancer Res. 48: 1596-1602; Reiss, M. & C.L. Dibble (1988) In Vitro Cell. Dev. Biol. 24:537-544) stimulation of growth of AKR-2B fibroblasts (Tucker, R.F., M.E. Olkenant, E.L. Branum & H.L. Moses (1988) Cancer Res. 43:1581-1586) and normal rat kidney fibroblasts, (Roberts, A.B., M.A. Anzano, L.C. Lamb, J.M. Smith & M.B. Sporn (1981) Proc. Natl. Acad. Sci. USA 78:5339-5343) stimulation of synthesis and secretion of fibronectin and collagen, (Ignotz, R. A. & J. Massague (1986) J. Biol. Chem. 261:4337-4345; Centrella, M., T.L. McCarthy & E. Canalis, (1987) J. Biol. Chem. 262:2869-2874) induction of cartilage-specific macromolecule production in muscle mesenchymal cells, (Seyedin, S. M., A. Y. Thompson, H. Bentz, D.M. Rosen, J. McPherson, A. Contin, N.R. Siegel, G.R. Galluppi & K.A. Piez (1986) J. Biol. Chem. 261:5693-5695) and growth inhibition of T and B lymphocytes. (Kehrl, J.H., L.M. Wakefiled, A.B. Roberts, S. Jakeoview, M. Alvarez-Mon, R. Derynck, M.B. Sporn & A.S. Fauci (1986) J. Exp. Med. 163:1037-1050; Kehrl, J.H., A.B. Roberts, L.M. Wakefield, S. Jakoview, M.B. Sporn & A.S. Fauci (1987) J. Immunol. 137:3855-3860; Kasid, A., G.I. Bell & E.P. Director, (1988) J. Immunol. 141:690-698; Wahl, S.M., D.A. Hunt, H.L. Wong, S. Dougherty, N. McCartney-Francis, L.M. Wahl, L. Ellingsworth, J.A. Schmidt, G. Hall, A.B. Roberts & M.B. Sporn (1988) J. Immunol. 140:3026-3032)

Recent investigations have indicad that TGF-β1 is a member of a family of closely related growth-modulating proteins including TGF-β2, (Seyedin, S.M., P.R. Segarini, D.M. Rosen, A.Y. Thompson, H. Bentz & J. Graycar (1987) J. Biol. Chem. 262:1946-1949; Cheifetz, S., J.A. Weatherbee, M.L.-S. Tsang, J.K. Anderson, J.E. Mole, R. Lucas & J. Massague (1987) Cell 48:409-415; Ikeda, T., M.M. Lioubin & H. Marquardt (1987) Biochemistry 26:2406-2410) TGF-β3, (TenDijke, P., P. Hansen, K. Iwata, C. Pieler & J.G. Foulkes (1988) Proc. Natl. Acad. Sci. USA 85:4715-4719; Derynck, R., P. Lindquist, A. Lee, D. Wen, J. Tamm, J.L. Graycar, L Rhee, A.J. Mason, D.A. Miller, R.J. Coffey, H.L. Moses & E.Y. Chen (1988) EMBO J. 7:3737-3743; Jakowlew, S.B., P.J. Dillard, P. Kondaiah, M.B. Spom & A.B. Roberts (1988) Mol. Endocrinology. 2: 747-755) TGF-β4, (Jakowlew, S. B., P. J. Dillard, M. B. Sporn & A.B. Roberts (1988) Mol. Endocrinology. 2:1186-1195) Mullerian inhibitory substance, (Cate, R.L., R.J. Mattaliano, C. Hession, R. Tizard, N.M. Faber, A. Cheung, E.G. Ninfa, A.Z. Frey, D.J. Dash, E.P. Chow, R.A. Fisher, J.M. Bertonis, G. Torres, B.P. Wallner, K.L. Ramachandran, R.C. Ragin, T.F. Manganaro, D.T. Maclaughlin & P.K, Donahoe (1986) Cell 45:685-698) and the inhibins. (Mason, A. J., J.S. Hayflick, N. Ling, F. Esch, N. Ueno, S.-Y. Ying, R. Guillemin, H. Niall & P.H. Seeburg (1985) Nature 318:659-663)

TGF-β1 is a 24-kDa protein consisting of two identical disulfide-bonded 12 kD subunits. (Assoian, R.K., A. Komoriya, C.A. Meyers, D.M. Miller & M.B. Sporn (1983) J. Biol. Chem. 258:7155-7160; Frolik, C.A., L.L. Dart, C.A. Meyers, D.M. Miller & M.B. Sporn (1983) Proc. Natl. Acad. Sci. USA 80:3676-3680; Frolik, C.A., L.M. Wakefiled, D.M. Smith & M.B. Sporn (1984) J. Biol. Chem. 259:10995-11000) Analysis of cDNA clones coding for human, (Derynck, R., J.A. Jarrett, E.Y. Chem, D.H. Eaton, J.R. Bell, R.K. Assoian, A.B. Roberts, M.B. Sporn & D.V. Goeddel (1985) Nature 316:701-705) murine, (Derynck, R., J.A. Jarrett, E.Y. Chem, & D.V. Goeddel (1986) J. Biol. Chem. 261:4377-4379) and simian (Sharples, K., G.D. Plowman, T.M. Rose, D.R. Twardzik & A.F. Purchio (1987) DNA 6:239-244) TGF-β1 indicates that this protein is synthesized as a larger 390 amino acid pre-pro-TGF-β1 precursor; the carboxyl terminal 112 amino acid portion is then proteolytically cleaved to yield the TGF-β1 monomer.

The simian TGF-β1 cDNA clone has been expressed to high levels in Chinese hamster ovary (CHO) cells. Analysis of the proteins secreted by these cells using sitespecific antipeptide antibodies, peptide mapping, and protein sequencing revealed that both mature and precursor forms of TGF-β were produced and were held together, in part, by a complex array of disulfide bonds. (Gentry, L.E., N.R. Webb, J. Lim, A. M. Brunner, J.E. Ranchalis, D.R. Twardzik, M.N. Lioubin, H. Marquardt & A.F. Purchio (1987) Mol. Cell Biol. 7:3418-3427; Gentry, L.E., M.N. Lioubin, A.F. Purchio & H. Marquardt (1988) Mol. Cell. Biol. 8:4162-4168) Upon purification away from the 24kD mature rTGF-β1, the 90 to 110 kD precursor complex was found to consist of three species: pro-TGF-β1, the pro-region of the TGF-β1 precursor, and mature TGF-β1. (Gentry, L.E., N.R. Webb, J. Lim, A.M. Brunner, J.E. Ranchalis, D.R. Twardzik, M.N. Lioubin, H. Marquardt & A.F. Purchio (1987) Mol. Cell Biol. 7:3418-3427; Gentry, L.E., M.N. Lioubin, A.F. Purchio & H. Marquardt (1988) Mol. Cell. Biol. 8:4162-4168) Detection of optimal biological activity required acidification before analysis, indicating that rTGF-β1 was secreted in a latent form.

The pro-region of the TGF-β1 precursor was found to be glycosylated at three sites (Asn 82, Asn 136, and Asn 176) and the first two of these (Asn 82 and Asn 136) contain mannose-6-phosphate residues. (Brunner, A.M., L.E. Gentry, J.A. Cooper & A.F. Purchio (1988) Mol. Cell Biol. 8:2229-2232; Purchio, A.F., J.A. Cooper, A.M. Brunner, M.N. Lioubin, L.E. Gentry, K.S. Kovacina, R.A. Roth & H. Marquardt. (1988) J. Biol. Chem. 263:14211-14215) In addition, the rTGF-β1 precursor is capable of binding to the mannose-6-phosphate receptor and may imply a mechanism for delivery to lysomes where proteolytic processing can occur. (Kornfeld, S. (1986) J. Clin. Ivest. 77:1-6)

TGF-β2 is also a 24-kD homodimer of identical disulfide-bonded 112 amino acid subunits (Marquardt, H., M.N. Lioubin & T. Ikeda (1987) J. Biol. Chem. 262:12127-12131). Analysis of cDNA clones coding for human (Madisen, L., N. R. Webb, T.M. Rose, H. Marquardt, T. Ikeda, D. Twardzik, S. Seyedin & A.F. Purchio. (1988) DNA 7:1-8; DeMartin, R., B. Plaendler, R. Hoefer-Warbinek, H. Gaugitsch, M. Wrann, H. Schlusener, J.M. Seifert, S. Bodmer, A. Fontana & E. Hoefer. EMBO J. 6:3673-3677) and simian (Hanks, S.K., R. Armour, J.H. Baldwin, F. Maldonado, J. Spiess & R.W. Holley (1988) Proc. Natl. Acad. Sci. USA 85:79-82) TGF-β2 showed that it, too, is synthesized as a larger precursor protein. The mature regions of TGF-β1 and TGF-β2 show 70% homology, whereas 30% homology occurs in the proregion of the precursor. In the case of simian and human TGF-β2 precursor proteins differing by a 28 amino acid insertion in the pro-region; mRNA coding for these two proteins is thought to occur via differential splicing (Webb, N.R., L. Madisen, T.M. Rose & A.F. Purchio (1988) DNA 7:493-497).

## SUMMARY OF THE INVENTION

The present invention is directed to the induction in mammalian cells of a new family of genes in response to TGF-beta administration. The induced genes encode a class of similar proteins containing about 345 to about 380 amino acid residues, having a molecular weight of about 37,000 daltons to about 45,000 daltons and containing about 38 cysteine residues. The cysteine residues are substantially conserved and these proteins share about 50% homology with each other. The induced gene products further share extensive homology with a protein induced by v-src in chicken embryo fibroblasts.

The present invention specifically discloses the induction by TGF-beta in mouse embryo cells of a gene family encoding proteins designated as βIG-M1 and βIG-M2 (beta-induced gene-mouse 1 and 2, respectively) that share about 80% and 50% homology, respectively with the CEF-10 protein induced by v-src in chicken embryo fibroblasts. The nucleotide sequences for βIG-M1 and βIG-M2 were elucidated and compared. The induction of the genes of the present invention by TGF-beta had not been previously reported or envisioned.

## DESCRIPTION OF THE FIGURES

In the drawings:

FIGURE 1 illustrates the nucleotide and deduced amino acid sequences of βIG-M1, and corresponds to Sequence I.D. No. 1.

FIGURE 2 illustrates the nucleotide and deduced amino acid sequences of βIG-M2, and corresponds to Sequence I.D. No. 3.

FIGURE 3 illustrates Northern Blot Analysis of βIG-M1 and βIG-M2 RNA. Total RNA was extracted from AKR-2B cells (Purchio and Fareed (1979) J. Virol. 29:763-769), fractionated on a 1% agarose-formaldehyde gel (Lehrach et al., ( 1977) Biochemistry 16:4743-4751 ) and hybridized to [$^{32}$P]-labelled βIG-M1 (A) or βIG-M2 (C) probes. Lane 1, AKR-2B; Lane 2, AKR-2B and TGF-β1; Lane 3, AKR-2B and cyclohexamide; Lane 4, AKR-2B and cyclohexamide and TGF-β1. The gels shown in panels A and C were stained with methylene blue and photographed (B and D) to show equal loading of RNAs.

FIGURE 4 illustrates the alignment of amino acid residue sequences for βIG-M1 and CEF-10 proteins. Residues that are identical in both sequences are indicated by (:).

FIGURE 5 illustrates the alignment of amino acid residue sequences for βIG-M2 and CEF-10 proteins. Residues that are identical in both sequences are indicated by (:).

FIGURE 6 illustrates the alignment of amino acid residue sequences for βIG-M2 and βIG-M1 proteins. Residues that are identical in both sequences are indicated by (:).

FIGURE 7 illustrates the multiple sequence alignment of region II of CS protein. The alignment shown is between 8 protein sequences. An asterisk (*) indicated the positions where alignment is perfectly conserved, and a dot (.) indicates those positions that are well conserved.

The aligned regions represented are:

. βIG-M1: amino acid residues 227-286 (60 residues)
. CEF12CS (CEF10): amino acid residues 224-283 (60 residues)
. βIG-M2: amino acid residues 198-257 (60 residues)
. PFALCIPACS (P. Falciparum CS protein region II): amino acid residues 340-395 (55 residues)
. PROPERDCSR (Properdin): consensus of 6 repots (60 residues)
. THROMBOCS (Trombospondin): repeat region, amino acid residues 420-476 (56 residues)
. PFALTRAPCS (P. Falciparum TRAP): amino acid residues 244-291 (48 residues)
. C7COMPCS (C7 terminal complement motif): amino acid residues 8-63 (56 residues)

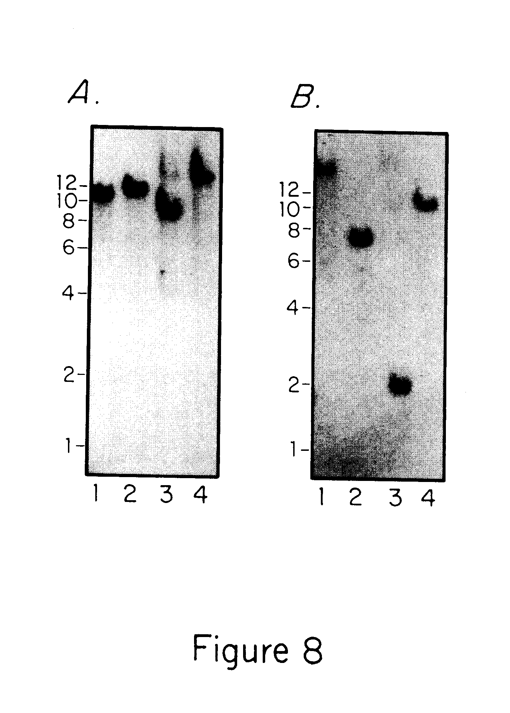

FIGURE 8 illustrates a Southern blot analysis of mouse genomic DNA with pβIG-M2. High molecular weight DNA was extracted from mouse kidneys, digested with Bam HI (lane 1), Eco RI (lane 2), Hind III (lane 3) or SstI (lane 4) and analyzed by Southern blotting with [$^{32}P$]-labeled pβIG-M2 (panel A) or [$^{32}P$]-labeled pβIG-M1 (panel B).

## DESCRIPTION OF PREFERRED EMBODIMENTS

The present invention is directed to the induction of a gene family by TGF-beta administration to target cells. The genes encode a family of proteins having about 345 to about 380 amino acid residues, having a molecular weight of about 37,000 daltons to about 45,000 daltons and containing about 38 cysteine residues.

TGF-β1 is known to regulate the transcription of several genes, such as the genes encoding c-myc, c-sis, the receptor for platelet derived growth factor (PDGF) and TGF-betal. The proteins encoded by the TGF-betal induced genes are likely involved in mediation of the biological effects of TGF-betal relating to cell growth and differentiation.

All amino acid residues identified herein are in the natural of L-configuration. In keeping with standard polypeptide nomenclature, abbreviations for amino acid residues are as follows:

| AMINO ACID | SYMBOL 3-Letter | SYMBOL 1-Letter |
|---|---|---|
| Alanine | Ala | A |
| Arginine | Arg | R |
| Asparagine | Asn | N |
| Aspartic acid | Asp | D |
| Aspartic acid or Asparagine | Asx | B |
| Cysteine | Cys | C |
| Glutamine | Gln | Q |
| Glutamic acid | Glu | E |
| Glycine | Gly | G |
| Glutamic acid or Glutamine | Glx | Z |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

In the present invention it was found that when cells are treated with TGF-betal, at least one new class of genes was transcriptionally activated. This class of genes was established by isolating the RNA from the treated cells, processing it, and then preparing cDNA from the RNA. The cDNA was further cloned and a library of genes prepared.

As used herein, the term "library" refers to a large random collection of cloned DNA fragments obtained from the transcription system of interest. The gene library was then screened with labelled cDNA probes obtained from TGF-beta treated and untreated cells. This approach led to the detection of TGF-betal induced genes.

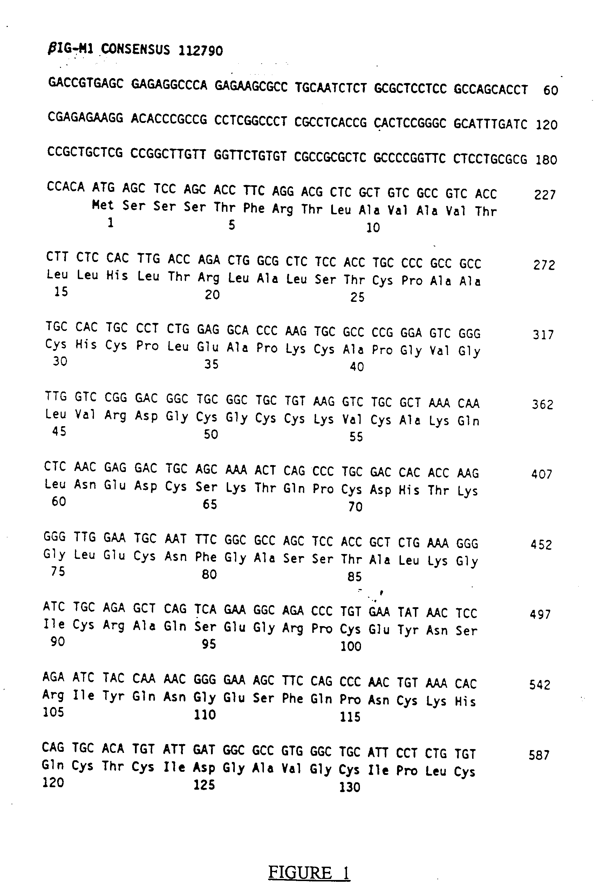

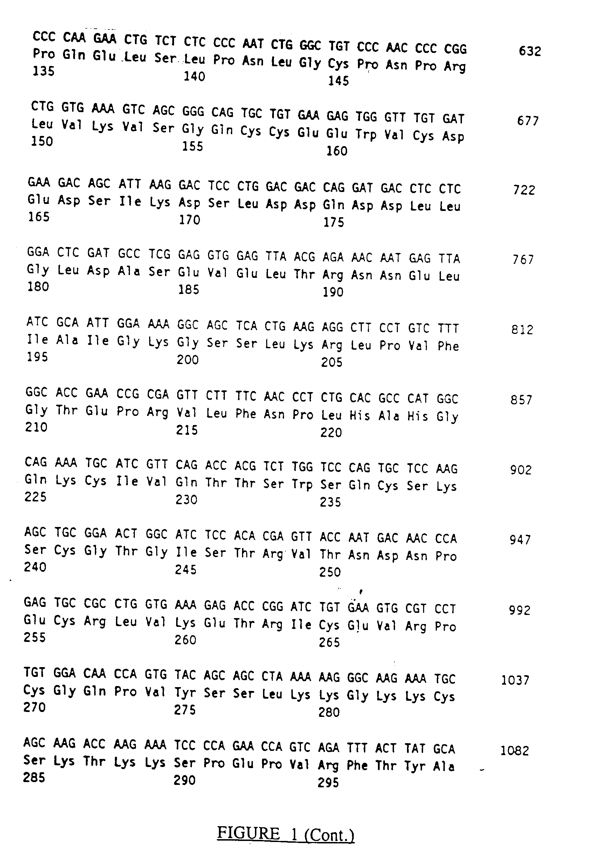

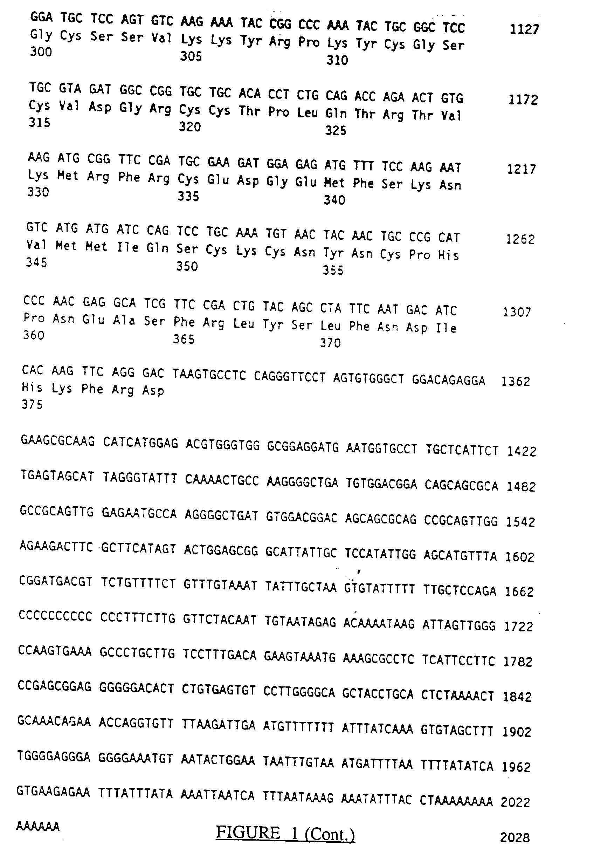

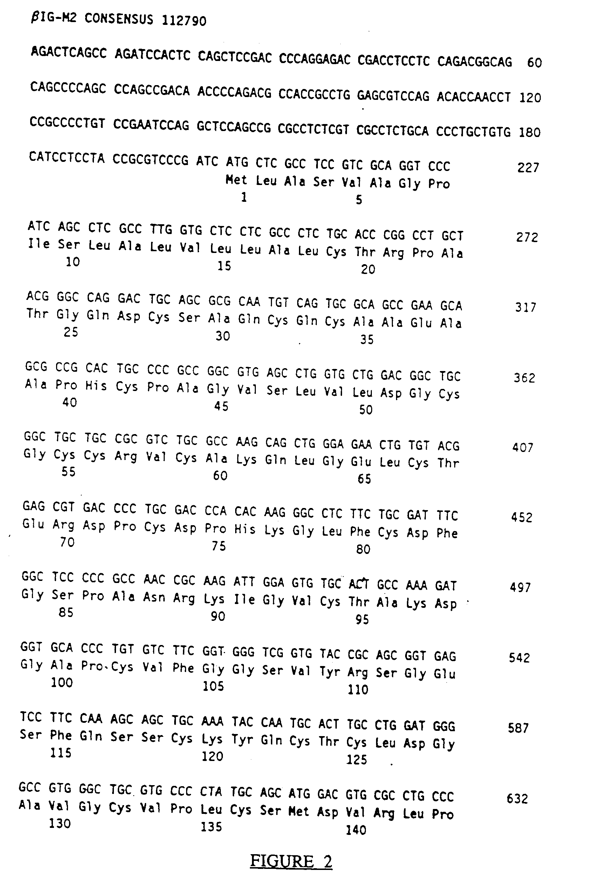

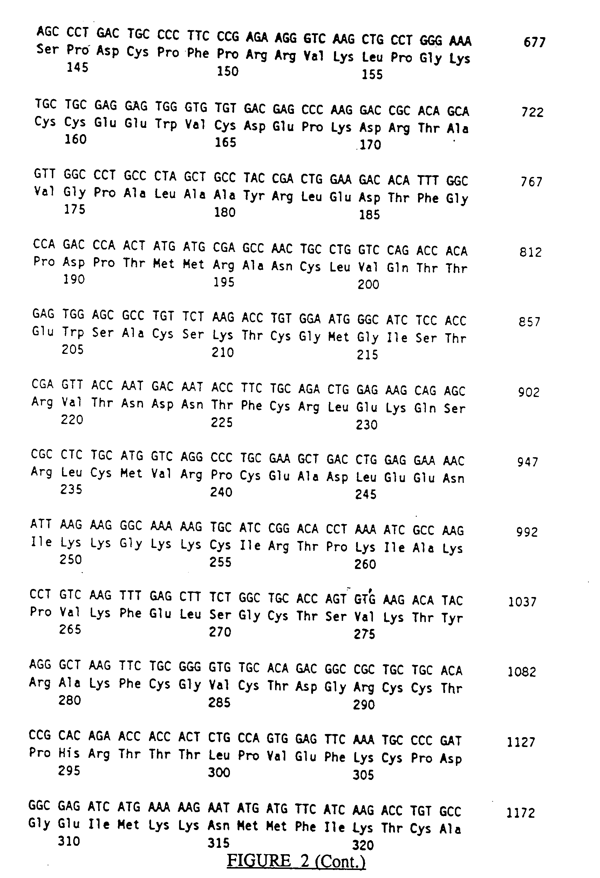

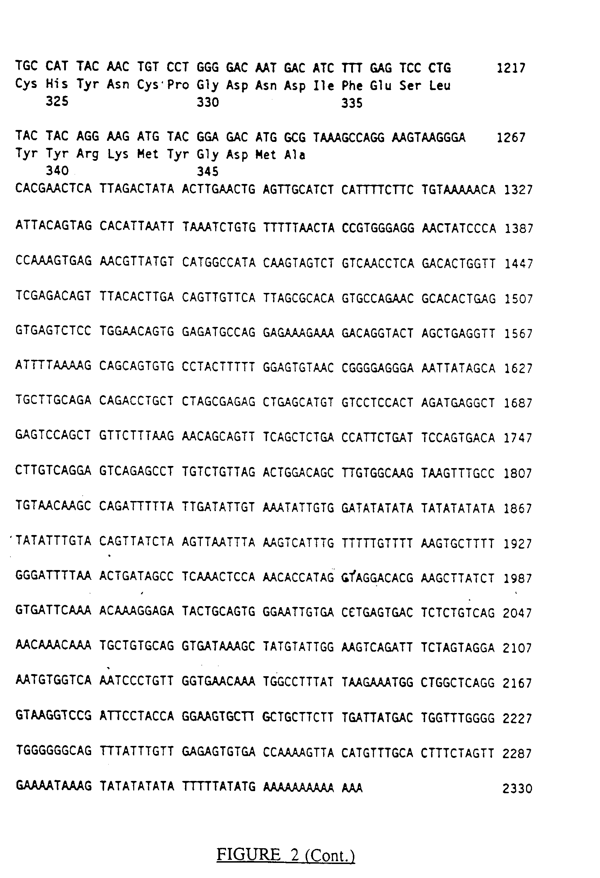

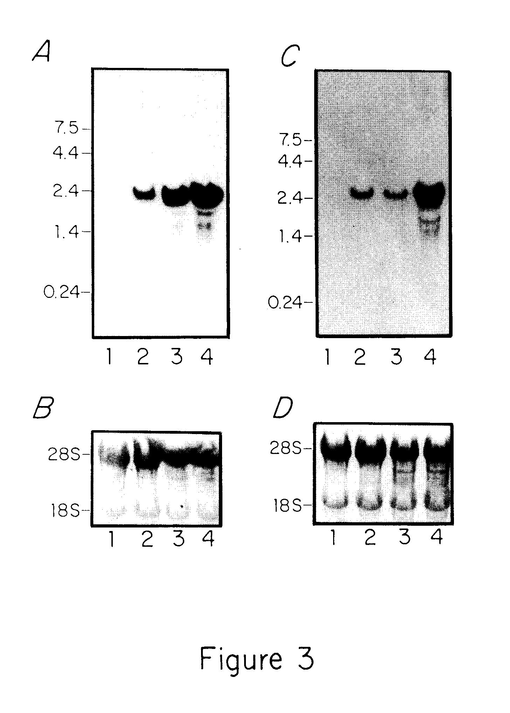

In a preferred embodiment, mouse AKR-2B cells (obtained from Dr. H. Moses, Vanderbilt University, Nashville, TN. ) were treated with TGF-beta1, and two new genes, designated βIG-M1 and βIG-M2, respectively, were elucidated. The coding sequences for these genes were obtained by cDNA cloning of the polyadenylated RNA isolated from the AKR-2B cells. The entire coding region was sequenced and then compared to known published sequences. The deduced amino acid sequences of the βIG-M1 and βIG-M2 gene products demonstrated about 80% and 50% homology, respectively, with CEF-10, a gene induced by v-src in chicken embryo fibroblasts (Simmons et al. (1989) Proc. Natl.. Acad. Sci. USA. 86:1178). Comparison and alignment of the amino acid sequences of CEF-10 with βIG-M1 and βIG-M2 are shown in FIGURES 1 and 2, respectively. It is readily seen that significant homology exists between these proteins and that 38 of the 39 cysteine residues are conserved. When βIG-M1 and βIG-M2 are compared with each other, approximately 50% homology is seen between the two sequences. (FIGURE 3)

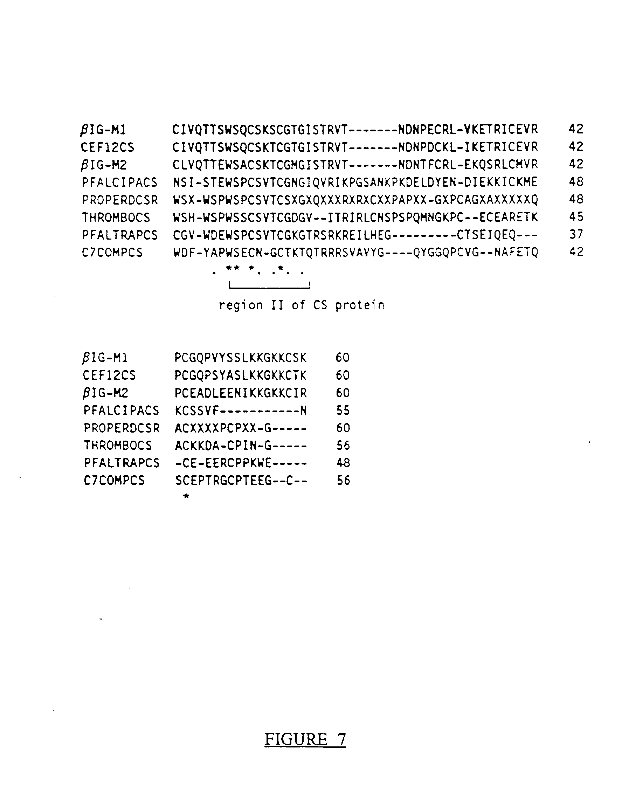

Upon further investigation it was found that the C-terminal cysteine rich domain of CEF-10, βIG-M1, and βIG-M2 contain an amino acid sequence motif with strong homology (9 of 12 amino acids) to a motif found near the C-terminal of the malarial circumsporozoite (CS) protein. (FIGURE 7) This region of the CS protein, designated 'region II', is highly conserved (10 of 12 amino acids) among all species of malarial parasites sequenced to date (Robson, K.J.H., et al. (1988) Nature 335:79; Rich, K.A., et al. (1990) Science 249:1574). The CS protein is expressed on the surface of plasmodium species during the sporozoite phase and may be involved in recognition and entry into hepatocytes (Aley, S.B., et al. (1986) J. Exp. Med. 164:1915).

The role of the region II motif in cell adhesion has been demonstrated by using peptide fragments of P.vivax CS protein to promote T-cell and myeloid cell line attachment to microtiter plates (Rich, K. A., et al. ( 1990) Science 249:1574). Furthermore, only peptides overlapping region II were able to inhibit T-cell and myeloid cell lines from binding to the CS protein.

The region II CS protein motif (CS motif is also found in other proteins which may have cell adhesive properties that mediate cell-cell and cell-extracellular matrix interactions, such as properdin, thrombospondin; thrombospondon related anonymous protein (TRAP) and various complement components.

Properdin has 6 repeats containing the CS motif. Properdin is involved in stabilizing the 'alternate' pathway of complement which involves the binding of C3b to the surfaces of foreign organisms (Goundis, D. and Reid, K.B.M. (1988) Nature 335:82).

Thrombospondin has 3 repeats of the CS motif. Data suggest it is a member of a class of adhesive proteins secreted by activated platelets and tissue culture cells, associating with the platelet membrane and becoming incorporated in fibrin clots and extracellular matrix (Lawler, J. and Hynes, R.O. (1986) J. Cell Bio. 103:1635).

TRAP is a surface antigen expressed during the blood stage of P. falciparum and may be involved in attachment to erythrocytes (possibly via C3b) prior to invasion (Robson, K.J.H., et al. (1988) Nature 335:79).

A comparison of the amino acid residue sequences of these proteins is shown in FIGURE 7, and demonstrates a high degree to conservation of the region II sequence.

The N-terminus and the C-terminus of complement components C7, C8α, and C8β, and the N-terminus of C9 contain motifs that have weak homology to the CS motif (Goundis, D. and Reid, K.B.M. (1988) Nature 335:82).

Libraries of cDNA were generated in the present invention as a means to detect the induction of new genes by TGF-beta1. Double stranded cDNA containing EcoR1 cohesive termini was ligated into the unique Ecol cloning site present in λ gt 10 DNA. The recombinant DNA was then packaged into viable phage particles and plated on appropriate hosts (E. coli strain $C_{600}$ $rK^{-}mK^{+}hFl$) for amplification and screening.

λ gt 10 is an insertion vector with a cloning capacity of up to 7 kb. The unique EcoR1 cloning site is located in the λ repressor (cI) gene. Insertion of foreign DNA at this restriction site interrupts the cI coding sequence and causes the phenotype of the phage to change from $cI^{+}$ (wild type) to $cI^{-}$. Since $cI^{-}$ phage are unable to lysogenize the host, clear plaques are produced by the recombinants. When plated on mutant bacteria which produce lysogeny, or bacteriophage integration, at a high frequency, only recombinant $cI^{-}$ phage produce plaques. Nonrecombinants, such as λ gt 10 without an insert, are effectively suppressed from plaque formation. This has served in the present invention as the basis for the biological selection for recombinant phage during λ gt 10 library amplification.

Selection of the cloned sequences of interest in the present invention was carried out by screening the library with nucleic acid sequences derived from TGF-β1 treated and untreated cells. This screening is dependent upon molecular hybridization by annealing of single-stranded nucleic acid molecules to form duplex structures that are stabilized by sequence-specific hydrogen bonds. Only nucleic acids of related sequence organization will base pair, or hybridize, with each other.

Northern blot analysis as carried out in the present invention allows the detection of rare RNA molecules in a cell. In this technique, total cellular RNA is prepared and then resolved into different size classes electrophoretically. The resolved RNA is then transferred and probed with radiolabelled DNA, followed by radioautographic detection of DNA-RNA hybrid duplexes.

The Northern blot technology was used in the present invention to further characterize βIG-M1 and βIG-M2.

The present invention is further described by the following Examples which are intended to be illustrative and not limiting.

EXAMPLE 1

Isolation of βIG-M1 and βIG-M2

AKR-2B mouse cells, (obtained from Dr. H. Moses, Vanderbilt University, Nashville, TN.) were grown to confluency in McCoy's media (GIBCO BRL, Gaithersburg, MD) plus 5% fetal bovine serum (FBS). The cells were then treated with cyclohexamide (10 ug/ml) for 15 minutes.

TGF-beta1 (10 ng/ml) was then added to the cells and the cells maintained for 6 hours at about 37°C with cyclohexamide and TGF-beta1.

The RNA was extracted from the cells. Polyadenylated RNA (polyA-RNA) was isolated by passage of the extracted RNA through an oligo-dT cellulose column. The polyA-RNA was then used to prepare cDNA by use of reverse transcriptase. The cDNA was cloned into λ gt 10 phage by using an EcoRI bridger according to the method of Webb, N.R. et al., 1987, DNA 6:71-79.

A DNA library was prepared and was then screened using two $^{32}P$-labelled cDNA probes. The $^{32}P$-labelled cDNA probes were prepared, respectively, from untreated AKR-2B mRNA and AKR-2B mRNA from cells treated with cyclohexamide and TGF-beta1. Hybridization of the probes with the DNA library to elicit plaques was carried out. Those plaques that had hybridized strongly with the probe from treated cells were isolated and further purified. The DNA from the tertiary plaques were cut with EcoR1 and then cloned into plasmid pEMBL18. Two clones (βIG-M1 and βIG-M2) were then sequenced. The sequences are shown in FIGURE 1 and 2 (Sequence I.D. Nos. 1 and 3, respectively).

Northern blot analysis of the mRNA from treated and untreated cells are shown in FIGURE 3. βIG-M1 (Figure 3A, lane 2) and βIG-M2 (Figure 3C, lane 2) RNAs were significantly increased in AKR-2B cells after a 6 hour treatment with TGF-β1. These RNA were barely detectable in untreated cells (Figures 3A and 3C, lane 1). Both βIG-M1 and βIG-M2 RNAs were increased by treatment with cyclohexamide alone (FIGURES 3A and 3C, lane 3) and were even further induced by treatment with the combination of cyclohexaminde and TGF-β1. (FIGURES 3A and 3C, lane 4). TGF-β1 treatment in the presence of cyclohexamide increased βIG-M2 RNA to a much higher extent (15 fold) than βIG-M1 RNA (3 fold) over those values observed after cyclohexamide treatment alone.

Southern blot analysis was carried out using mouse kidney DNA and clearly demonstrated that the two probes hybridized to different restriction fragments (FIGURE 8A and B) indicating that βIG-M1 and βIG-M2 are encoded by different genes. It is readily seen that the administration of TGF-β1 in the presence of cyclohexamide significantly induces the production of mRNA for both βIG-M1 and βIG-M2 (FIGURE 3). A small amount of constitutive synthesis of these mRNAs is seen in the cyclohexamide treated cells.

EXAMPLE 2

Characterization of βIG-M1 and βIG-M2

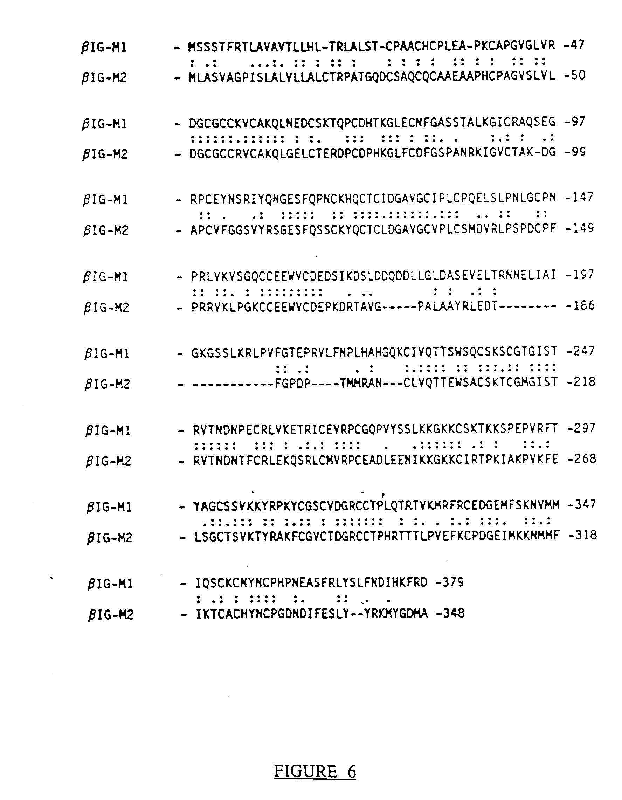

The amino acid residue sequences for βIG-M1 and βIG-M2 (sequence I.D. No. 2 and 4, respectively) were determined and compared. As shown in FIGURE 6 when the two protein sequences are aligned there is a 47.7% homology between the sequences with conservation of 38 of the 39 cysteine residues.

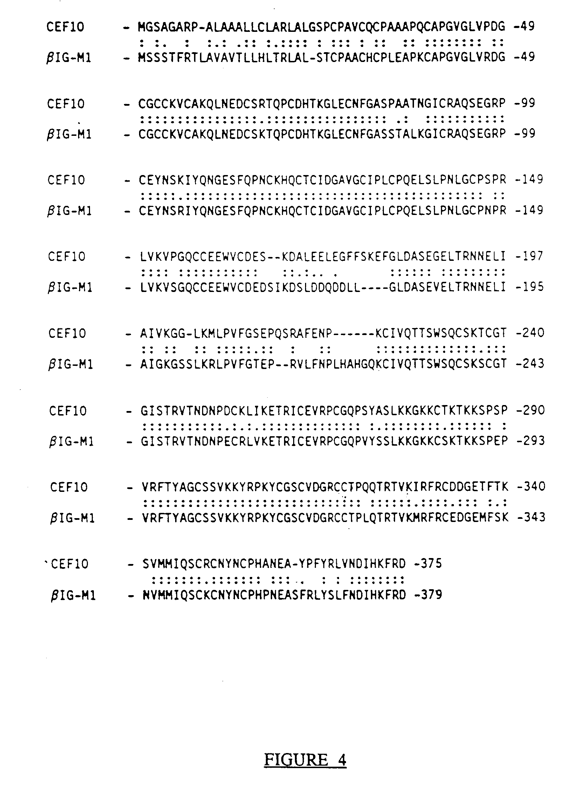

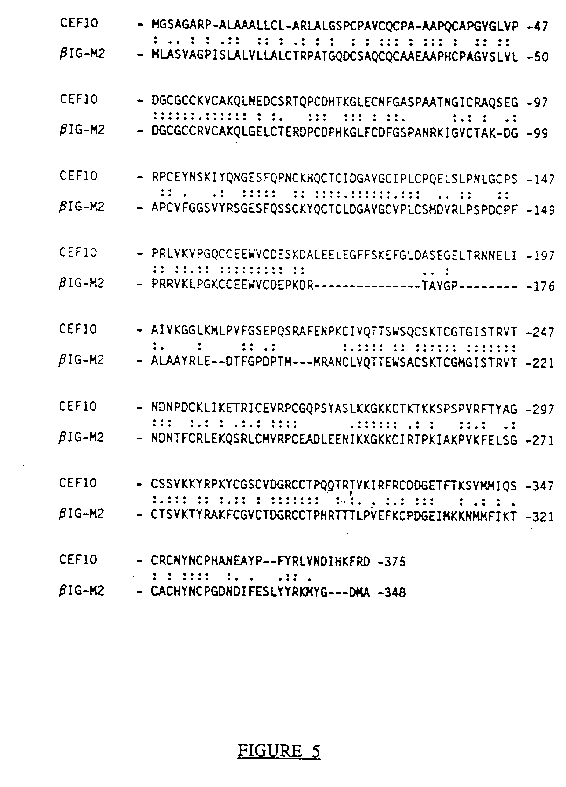

Comparison of the protein sequence with the v-src-induced gene product CEF-10 (Sequence I.D. No. 6) shows homology of about 80% with βIG-M1 (Sequence I.D. No. 2) as seen in FIGURE 4, and of about 50% with βIG-M2 (Sequence I.D. No. 4) as seen in FIGURE 5.

DNA sequence analysis of pβIG-M1 indicated that it contained a single open reading frame coding for a 379 amino acid polypeptide. As stated above, this protein is about 80% homologous to CEF-10. It was further determined that βIG-M1 protein is identical to the protein encoded by cyr61, as described in O'Brien et al. (1990) Mol. Cell Biol. 10:3569-3577, an immediate early response gene induced in quiescent BALB 3T3 cells by serum treatment.

DNA sequence analysis of pβIG-M2 (FIGURE 2) indicates a single open reading frame encoding a 348 amino acid protein. The amino terminal portion of βIG-M2 contains a hydrophobic stretch which could function as a signal peptide. Beginning at amino acid residue 52 in FIGURE 2, βIG-M2 contains the sequence Gly-Cys-Gly-Cys-Cys-Arg-Val-Cys which conforms to the Gly-Cys-Gly-Cys-Cys-X-X-Cys motif reported in the amino half of insulin-like growth factor (IGF) binding proteins. (Binkert et al. (1988) EMBO J. 8:2497-2502; Albiston et al. (1990) Biochem. Biophys. Res. Commun. 16:892-897; Brinkman et al. (1988) EMBO J. 7:2417-2423). This motif is also present in βIG-M1 at amino acid residues 49 - 56 in Figure 1.

The foregoing description and Examples are intended as illustrative of the present invention, but not as limiting. Numerous variations and modifications may be effected without departing from the true spirit and scope of the present invention.

## Claims

1. A substantially purified protein comprising about 345 to about 380 amino acid residues, having a molecular weight of about 37,000 daltons to about 45,000 daltons and containing about 38 cysteine residues, said protein being induced by TGF-beta administration to mammalian cells.

2. The protein according to Claim 1, wherein the protein has an amino acid residue sequence substantially corresponding to the sequence depicted in FIGURE 1 designated as βIG-M1 and having Sequence I.D. No. 2.

3. The protein according to Claim 1, wherein the protein has an amino acid residue sequence substantially corresponding to the sequence depicted in FIGURE 2 designated as βIG-M2 and having Sequence I.D. No. 4.

4. The protein according to Claim 2 encoded by a nucleotide sequence substantially corresponding to the sequence of FIGURE 1 and having Sequence I.D. No. 1.

5. The protein according to Claim 3 encoded by a nucleotide sequence substantially corresponding to the sequence of FIGURE 2 and having Sequence I.D. No. 3.

6. A nucleotide sequence encoding a TGF-beta induced protein substantially corresponding to the nucleotide sequence depicted in FIGURE 1 and having Sequence I.D. No. 1.

7. A nucleotide sequence encoding a TGF-beta-induced protein substantially corresponding to the nucleotide sequence depicted in FIGURE 2 and having Sequence I.D. No. 3.

8. A gene family induced by TGF-beta wherein the induced genes encode a protein comprising about 345 to about 380 amino acid residues, having a molecular weight of about 37,000 daltons to about 45,000 daltons and containing about 38 cysteine residues.

9. The gene family according to Claim 8 wherein an induced gene encodes a protein having an amino acid residue sequence substantially corresponding to the sequence depicted in FIGS 1 and having Sequence I.D. No. 2.

10. The gene family according to Claim 8 wherein an induced gene encodes a protein having an amino acid residue sequence substantially corresponding to the sequence depicted in FIGS 2 and having Sequence I.D. No. 4.

11. The gene family according to Claim 8 wherein an induced gene has a nucleotide sequence substantially corresponding to the sequence depicted in FIGURE 1 and having Sequence I.D. No. 1.

**12.** The gene family according to Claim 8 wherein an induced gene has a nucleotide sequence substantially corresponding to the sequence depicted in FIGURE 2 and having Sequence I.D. No. 3.

**13.** A method for the determination of a TGF-β induced gene comprising the steps of:
(1) treating a mammalian cell with an effective amount of an inhibitor of mRNA translation for a time period sufficient to inhibit protein synthesis;
(2) further treating said mammalian cell with an effective amount of TGF-β for a time period sufficient to induce mRNA synthesis from TGF-β inducible genes;
(3) preparing a cDNA library from mRNA isolated from the cell treated according to steps (1) and (2);
(4) probing the cDNA library with cDNA isolated from the untreated mammalian cell of step (1);
(5) probing the cDNA library with cDNA isolated from the mammalian cell treated according to steps (1) and (2);
(6) selecting a cDNA detectted in step (4) but not in step (5); and
(7) sequencing the DNA selected in step (6).

**14.** A method for the production of a protein according to any one of claims 1 to 5 comprising the steps of:
(1) inserting a nucleic acid coding sequence encoding the protein into an expression vector;
(2) transforming or transfecting a mammalian cell with the expression vector;
(3) culturing the mammalian cell to express the protein; and
(4) isolating the protein.

βIG-M1 CONSENSUS 112790

```
GACCGTGAGC GAGAGGCCCA GAGAAGCGCC TGCAATCTCT GCGCTCCTCC GCCAGCACCT  60

CGAGAGAAGG ACACCCGCCG CCTCGGCCCT CGCCTCACCG CACTCCGGGC GCATTTGATC 120

CCGCTGCTCG CCGGCTTGTT GGTTCTGTGT CGCCGCGCTC GCCCCGGTTC CTCCTGCGCG 180

CCACA ATG AGC TCC AGC ACC TTC AGG ACG CTC GCT GTC GCC GTC ACC     227
      Met Ser Ser Ser Thr Phe Arg Thr Leu Ala Val Ala Val Thr
       1               5                  10

CTT CTC CAC TTG ACC AGA CTG GCG CTC TCC ACC TGC CCC GCC GCC       272
Leu Leu His Leu Thr Arg Leu Ala Leu Ser Thr Cys Pro Ala Ala
 15                 20                  25

TGC CAC TGC CCT CTG GAG GCA CCC AAG TGC GCC CCG GGA GTC GGG       317
Cys His Cys Pro Leu Glu Ala Pro Lys Cys Ala Pro Gly Val Gly
 30                 35                  40

TTG GTC CGG GAC GGC TGC GGC TGC TGT AAG GTC TGC GCT AAA CAA       362
Leu Val Arg Asp Gly Cys Gly Cys Cys Lys Val Cys Ala Lys Gln
 45                 50                  55

CTC AAC GAG GAC TGC AGC AAA ACT CAG CCC TGC GAC CAC ACC AAG       407
Leu Asn Glu Asp Cys Ser Lys Thr Gln Pro Cys Asp His Thr Lys
 60                 65                  70

GGG TTG GAA TGC AAT TTC GGC GCC AGC TCC ACC GCT CTG AAA GGG       452
Gly Leu Glu Cys Asn Phe Gly Ala Ser Ser Thr Ala Leu Lys Gly
 75                 80                  85

ATC TGC AGA GCT CAG TCA GAA GGC AGA CCC TGT GAA TAT AAC TCC       497
Ile Cys Arg Ala Gln Ser Glu Gly Arg Pro Cys Glu Tyr Asn Ser
 90                 95                 100

AGA ATC TAC CAA AAC GGG GAA AGC TTC CAG CCC AAC TGT AAA CAC       542
Arg Ile Tyr Gln Asn Gly Glu Ser Phe Gln Pro Asn Cys Lys His
105                110                 115

CAG TGC ACA TGT ATT GAT GGC GCC GTG GGC TGC ATT CCT CTG TGT       587
Gln Cys Thr Cys Ile Asp Gly Ala Val Gly Cys Ile Pro Leu Cys
120                125                 130
```

FIGURE 1

CCC CAA GAA CTG TCT CTC CCC AAT CTG GGC TGT CCC AAC CCC CGG 632
Pro Gln Glu Leu Ser Leu Pro Asn Leu Gly Cys Pro Asn Pro Arg
135 140 145

CTG GTG AAA GTC AGC GGG CAG TGC TGT GAA GAG TGG GTT TGT GAT 677
Leu Val Lys Val Ser Gly Gln Cys Cys Glu Glu Trp Val Cys Asp
150 155 160

GAA GAC AGC ATT AAG GAC TCC CTG GAC GAC CAG GAT GAC CTC CTC 722
Glu Asp Ser Ile Lys Asp Ser Leu Asp Asp Gln Asp Asp Leu Leu
165 170 175

GGA CTC GAT GCC TCG GAG GTG GAG TTA ACG AGA AAC AAT GAG TTA 767
Gly Leu Asp Ala Ser Glu Val Glu Leu Thr Arg Asn Asn Glu Leu
180 185 190

ATC GCA ATT GGA AAA GGC AGC TCA CTG AAG AGG CTT CCT GTC TTT 812
Ile Ala Ile Gly Lys Gly Ser Ser Leu Lys Arg Leu Pro Val Phe
195 200 205

GGC ACC GAA CCG CGA GTT CTT TTC AAC CCT CTG CAC GCC CAT GGC 857
Gly Thr Glu Pro Arg Val Leu Phe Asn Pro Leu His Ala His Gly
210 215 220

CAG AAA TGC ATC GTT CAG ACC ACG TCT TGG TCC CAG TGC TCC AAG 902
Gln Lys Cys Ile Val Gln Thr Thr Ser Trp Ser Gln Cys Ser Lys
225 230 235

AGC TGC GGA ACT GGC ATC TCC ACA CGA GTT ACC AAT GAC AAC CCA 947
Ser Cys Gly Thr Gly Ile Ser Thr Arg Val Thr Asn Asp Asn Pro
240 245 250

GAG TGC CGC CTG GTG AAA GAG ACC CGG ATC TGT GAA GTG CGT CCT 992
Glu Cys Arg Leu Val Lys Glu Thr Arg Ile Cys Glu Val Arg Pro
255 260 265

TGT GGA CAA CCA GTG TAC AGC AGC CTA AAA AAG GGC AAG AAA TGC 1037
Cys Gly Gln Pro Val Tyr Ser Ser Leu Lys Lys Gly Lys Lys Cys
270 275 280

AGC AAG ACC AAG AAA TCC CCA GAA CCA GTC AGA TTT ACT TAT GCA 1082
Ser Lys Thr Lys Lys Ser Pro Glu Pro Val Arg Phe Thr Tyr Ala
285 290 295

FIGURE 1 (Cont.)

GGA TGC TCC AGT GTC AAG AAA TAC CGG CCC AAA TAC TGC GGC TCC 1127
Gly Cys Ser Ser Val Lys Lys Tyr Arg Pro Lys Tyr Cys Gly Ser
300 305 310

TGC GTA GAT GGC CGG TGC TGC ACA CCT CTG CAG ACC AGA ACT GTG 1172
Cys Val Asp Gly Arg Cys Cys Thr Pro Leu Gln Thr Arg Thr Val
315 320 325

AAG ATG CGG TTC CGA TGC GAA GAT GGA GAG ATG TTT TCC AAG AAT 1217
Lys Met Arg Phe Arg Cys Glu Asp Gly Glu Met Phe Ser Lys Asn
330 335 340

GTC ATG ATG ATC CAG TCC TGC AAA TGT AAC TAC AAC TGC CCG CAT 1262
Val Met Met Ile Gln Ser Cys Lys Cys Asn Tyr Asn Cys Pro His
345 350 355

CCC AAC GAG GCA TCG TTC CGA CTG TAC AGC CTA TTC AAT GAC ATC 1307
Pro Asn Glu Ala Ser Phe Arg Leu Tyr Ser Leu Phe Asn Asp Ile
360 365 370

CAC AAG TTC AGG GAC TAAGTGCCTC CAGGGTTCCT AGTGTGGGCT GGACAGAGGA 1362
His Lys Phe Arg Asp
375

GAAGCGCAAG CATCATGGAG ACGTGGGTGG GCGGAGGATG AATGGTGCCT TGCTCATTCT 1422

TGAGTAGCAT TAGGGTATTT CAAAACTGCC AAGGGGCTGA TGTGGACGGA CAGCAGCGCA 1482

GCCGCAGTTG GAGAATGCCA AGGGGCTGAT GTGGACGGAC AGCAGCGCAG CCGCAGTTGG 1542

AGAAGACTTC GCTTCATAGT ACTGGAGCGG GCATTATTGC TCCATATTGG AGCATGTTTA 1602

CGGATGACGT TCTGTTTTCT GTTTGTAAAT TATTTGCTAA GTGTATTTTT TTGCTCCAGA 1662

CCCCCCCCCC CCCTTTCTTG GTTCTACAAT TGTAATAGAG ACAAAATAAG ATTAGTTGGG 1722

CCAAGTGAAA GCCCTGCTTG TCCTTTGACA GAAGTAAATG AAAGCGCCTC TCATTCCTTC 1782

CCGAGCGGAG GGGGGACACT CTGTGAGTGT CCTTGGGGCA GCTACCTGCA CTCTAAAACT 1842

GCAAACAGAA ACCAGGTGTT TTAAGATTGA ATGTTTTTTT ATTTATCAAA GTGTAGCTTT 1902

TGGGGAGGGA GGGGAAATGT AATACTGGAA TAATTTGTAA ATGATTTTAA TTTTATATCA 1962

GTGAAGAGAA TTTATTTATA AAATTAATCA TTTAATAAAG AAATATTTAC CTAAAAAAAA 2022

AAAAAA 2028

FIGURE 1 (Cont.)

βIG-M2 CONSENSUS 112790

```
AGACTCAGCC AGATCCACTC CAGCTCCGAC CCCAGGAGAC CGACCTCCTC CAGACGGCAG   60

CAGCCCCAGC CCAGCCGACA ACCCCAGACG CCACCGCCTG GAGCGTCCAG ACACCAACCT 120

CCGCCCCTGT CCGAATCCAG GCTCCAGCCG CGCCTCTCGT CGCCTCTGCA CCCTGCTGTG 180

CATCCTCCTA CCGCGTCCCG ATC ATG CTC GCC TCC GTC GCA GGT CCC          227
                          Met Leu Ala Ser Val Ala Gly Pro
                           1               5

ATC AGC CTC GCC TTG GTG CTC CTC GCC CTC TGC ACC CGG CCT GCT        272
Ile Ser Leu Ala Leu Val Leu Leu Ala Leu Cys Thr Arg Pro Ala
    10                  15                  20

ACG GGC CAG GAC TGC AGC GCG CAA TGT CAG TGC GCA GCC GAA GCA        317
Thr Gly Gln Asp Cys Ser Ala Gln Cys Gln Cys Ala Ala Glu Ala
    25                  30                  35

GCG CCG CAC TGC CCC GCC GGC GTG AGC CTG GTG CTG GAC GGC TGC        362
Ala Pro His Cys Pro Ala Gly Val Ser Leu Val Leu Asp Gly Cys
    40                  45                  50

GGC TGC TGC CGC GTC TGC GCC AAG CAG CTG GGA GAA CTG TGT ACG        407
Gly Cys Cys Arg Val Cys Ala Lys Gln Leu Gly Glu Leu Cys Thr
    55                  60                  65

GAG CGT GAC CCC TGC GAC CCA CAC AAG GGC CTC TTC TGC GAT TTC        452
Glu Arg Asp Pro Cys Asp Pro His Lys Gly Leu Phe Cys Asp Phe
    70                  75                  80

GGC TCC CCC GCC AAC CGC AAG ATT GGA GTG TGC ACT GCC AAA GAT        497
Gly Ser Pro Ala Asn Arg Lys Ile Gly Val Cys Thr Ala Lys Asp
    85                  90                  95

GGT GCA CCC TGT GTC TTC GGT GGG TCG GTG TAC CGC AGC GGT GAG        542
Gly Ala Pro Cys Val Phe Gly Gly Ser Val Tyr Arg Ser Gly Glu
    100                 105                 110

TCC TTC CAA AGC AGC TGC AAA TAC CAA TGC ACT TGC CTG GAT GGG        587
Ser Phe Gln Ser Ser Cys Lys Tyr Gln Cys Thr Cys Leu Asp Gly
    115                 120                 125

GCC GTG GGC TGC GTG CCC CTA TGC AGC ATG GAC GTG CGC CTG CCC        632
Ala Val Gly Cys Val Pro Leu Cys Ser Met Asp Val Arg Leu Pro
    130                 135                 140
```

FIGURE 2

AGC CCT GAC TGC CCC TTC CCG AGA AGG GTC AAG CTG CCT GGG AAA 677
Ser Pro Asp Cys Pro Phe Pro Arg Arg Val Lys Leu Pro Gly Lys
145 150 155

TGC TGC GAG GAG TGG GTG TGT GAC GAG CCC AAG GAC CGC ACA GCA 722
Cys Cys Glu Glu Trp Val Cys Asp Glu Pro Lys Asp Arg Thr Ala
160 165 170

GTT GGC CCT GCC CTA GCT GCC TAC CGA CTG GAA GAC ACA TTT GGC 767
Val Gly Pro Ala Leu Ala Ala Tyr Arg Leu Glu Asp Thr Phe Gly
175 180 185

CCA GAC CCA ACT ATG ATG CGA GCC AAC TGC CTG GTC CAG ACC ACA 812
Pro Asp Pro Thr Met Met Arg Ala Asn Cys Leu Val Gln Thr Thr
190 195 200

GAG TGG AGC GCC TGT TCT AAG ACC TGT GGA ATG GGC ATC TCC ACC 857
Glu Trp Ser Ala Cys Ser Lys Thr Cys Gly Met Gly Ile Ser Thr
205 210 215

CGA GTT ACC AAT GAC AAT ACC TTC TGC AGA CTG GAG AAG CAG AGC 902
Arg Val Thr Asn Asp Asn Thr Phe Cys Arg Leu Glu Lys Gln Ser
220 225 230

CGC CTC TGC ATG GTC AGG CCC TGC GAA GCT GAC CTG GAG GAA AAC 947
Arg Leu Cys Met Val Arg Pro Cys Glu Ala Asp Leu Glu Glu Asn
235 240 245

ATT AAG AAG GGC AAA AAG TGC ATC CGG ACA CCT AAA ATC GCC AAG 992
Ile Lys Lys Gly Lys Lys Cys Ile Arg Thr Pro Lys Ile Ala Lys
250 255 260

CCT GTC AAG TTT GAG CTT TCT GGC TGC ACC AGT GTG AAG ACA TAC 1037
Pro Val Lys Phe Glu Leu Ser Gly Cys Thr Ser Val Lys Thr Tyr
265 270 275

AGG GCT AAG TTC TGC GGG GTG TGC ACA GAC GGC CGC TGC TGC ACA 1082
Arg Ala Lys Phe Cys Gly Val Cys Thr Asp Gly Arg Cys Cys Thr
280 285 290

CCG CAC AGA ACC ACC ACT CTG CCA GTG GAG TTC AAA TGC CCC GAT 1127
Pro His Arg Thr Thr Thr Leu Pro Val Glu Phe Lys Cys Pro Asp
295 300 305

GGC GAG ATC ATG AAA AAG AAT ATG ATG TTC ATC AAG ACC TGT GCC 1172
Gly Glu Ile Met Lys Lys Asn Met Met Phe Ile Lys Thr Cys Ala
310 315 320

FIGURE 2 (Cont.)

```
TGC CAT TAC AAC TGT CCT GGG GAC AAT GAC ATC TTT GAG TCC CTG       1217
Cys His Tyr Asn Cys Pro Gly Asp Asn Asp Ile Phe Glu Ser Leu
    325                 330                 335

TAC TAC AGG AAG ATG TAC GGA GAC ATG GCG TAAAGCCAGG AAGTAAGGGA     1267
Tyr Tyr Arg Lys Met Tyr Gly Asp Met Ala
    340                 345
CACGAACTCA TTAGACTATA ACTTGAACTG AGTTGCATCT CATTTTCTTC TGTAAAAACA 1327

ATTACAGTAG CACATTAATT TAAATCTGTG TTTTTAACTA CCGTGGGAGG AACTATCCCA 1387

CCAAAGTGAG AACGTTATGT CATGGCCATA CAAGTAGTCT GTCAACCTCA GACACTGGTT 1447

TCGAGACAGT TTACACTTGA CAGTTGTTCA TTAGCGCACA GTGCCAGAAC GCACACTGAG 1507

GTGAGTCTCC TGGAACAGTG GAGATGCCAG GAGAAAGAAA GACAGGTACT AGCTGAGGTT 1567

ATTTTAAAAG CAGCAGTGTG CCTACTTTTT GGAGTGTAAC CGGGGAGGGA AATTATAGCA 1627

TGCTTGCAGA CAGACCTGCT CTAGCGAGAG CTGAGCATGT GTCCTCCACT AGATGAGGCT 1687

GAGTCCAGCT GTTCTTTAAG AACAGCAGTT TCAGCTCTGA CCATTCTGAT TCCAGTGACA 1747

CTTGTCAGGA GTCAGAGCCT TGTCTGTTAG ACTGGACAGC TTGTGGCAAG TAAGTTTGCC 1807

TGTAACAAGC CAGATTTTTA TTGATATTGT AAATATTGTG GATATATATA TATATATATA 1867

TATATTTGTA CAGTTATCTA AGTTAATTTA AAGTCATTTG TTTTTGTTTT AAGTGCTTTT 1927

GGGATTTTAA ACTGATAGCC TCAAACTCCA AACACCATAG GTAGGACACG AAGCTTATCT 1987

GTGATTCAAA ACAAAGGAGA TACTGCAGTG GGAATTGTGA CCTGAGTGAC TCTCTGTCAG 2047

AACAAACAAA TGCTGTGCAG GTGATAAAGC TATGTATTGG AAGTCAGATT TCTAGTAGGA 2107

AATGTGGTCA AATCCCTGTT GGTGAACAAA TGGCCTTTAT TAAGAAATGG CTGGCTCAGG 2167

GTAAGGTCCG ATTCCTACCA GGAAGTGCTT GCTGCTTCTT TGATTATGAC TGGTTTGGGG 2227

TGGGGGGCAG TTTATTTGTT GAGAGTGTGA CCAAAAGTTA CATGTTTGCA CTTTCTAGTT 2287

GAAAATAAAG TATATATATA TTTTTATATG AAAAAAAAAA AAA                   2330
```

FIGURE 2 (Cont.)

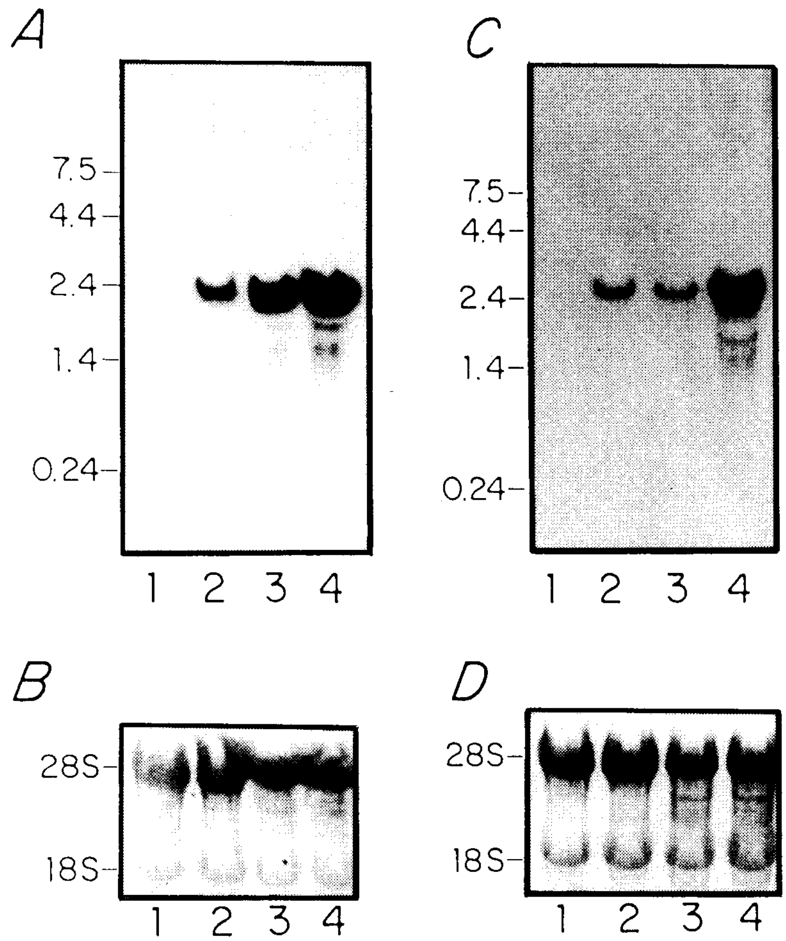
A
7.5
4.4
2.4
1.4
0.24
1 2 3 4
C
7.5
4.4
2.4
1.4
0.24
1 2 3 4
B
28S
18S
1 2 3 4
D
28S
18S
1 2 3 4

Figure 3

```
CEF10    - MGSAGARP-ALAAALLCLARLALGSPCPAVCQCPAAAPQCAPGVGLVPDG -49
           : :.  :  :.: .:: :.:::: : ::: : ::  :: :::::::: ::
βIG-M1   - MSSSTFRTLAVAVTLLHLTRLAL-STCPAACHCPLEAPKCAPGVGLVRDG -49

CEF10    - CGCCKVCAKQLNEDCSRTQPCDHTKGLECNFGASPAATNGICRAQSEGRP -99
           ::::::::::::::::.::::::::::::::::: .:  :::::::::::
βIG-M1   - CGCCKVCAKQLNEDCSKTQPCDHTKGLECNFGASSTALKGICRAQSEGRP -99

CEF10    - CEYNSKIYQNGESFQPNCKHQCTCIDGAVGCIPLCPQELSLPNLGCPSPR -149
           :::::.::::::::::::::::::::::::::::::::::::::::: ::
βIG-M1   - CEYNSRIYQNGESFQPNCKHQCTCIDGAVGCIPLCPQELSLPNLGCPNPR -149

CEF10    - LVKVPGQCCEEWVCDES--KDALEELEGFFSKEFGLDASEGELTRNNELI -197
           :::: :::::::::::   ::.:.. .       :::::: :::::::::
βIG-M1   - LVKVSGQCCEEWVCDEDSIKDSLDDQDDLL----GLDASEVELTRNNELI -195

CEF10    - AIVKGG-LKMLPVFGSEPQSRAFENP------KCIVQTTSWSQCSKTCGT -240
           :: ::  :: :::::.::  :   ::      ::::::::::::::.:::
βIG-M1   - AIGKGSSLKRLPVFGTEP--RVLFNPLHAHGQKCIVQTTSWSQCSKSCGT -243

CEF10    - GISTRVTNDNPDCKLIKETRICEVRPCGQPSYASLKKGKKCTKTKKSPSP -290
           :::::::::::.:.:.:::::::::::::: :.::::::::.:::::: :
βIG-M1   - GISTRVTNDNPECRLVKETRICEVRPCGQPVYSSLKKGKKCSKTKKSPEP -293

CEF10    - VRFTYAGCSSVKKYRPKYCGSCVDGRCCTPQQTRTVKIRFRCDDGETFTK -340
           :::::::::::::::::::::::::::::: ::::::.::::.::: :.:
βIG-M1   - VRFTYAGCSSVKKYRPKYCGSCVDGRCCTPLQTRTVKMRFRCEDGEMFSK -343

CEF10    - SVMMIQSCRCNYNCPHANEA-YPFYRLVNDIHKFRD -375
            :::::::.::::::: :::..  : : ::::::::
βIG-M1   - NVMMIQSCKCNYNCPHPNEASFRLYSLFNDIHKFRD -379
```

FIGURE 4

```
CEF10     - MGSAGARP-ALAAALLCL-ARLALGSPCPAVCQCPA-AAPQCAPGVGLVP -47
            : .. : : .::  :: : .: : :  : : ::: : ::: :  :: ::
βIG-M2    - MLASVAGPISLALVLLALCTRPATGQDCSAQCQCAAEAAPHCPAGVSLVL -50

CEF10     - DGCGCCKVCAKQLNEDCSRTQPCDHTKGLECNFGASPAATNGICRAQSEG -97
            ::::::.:::::: : :.   :::  ::: : ::.      :.: :  .:
βIG-M2    - DGCGCCRVCAKQLGELCTERDPCDPHKGLFCDFGSPANRKIGVCTAK-DG -99

CEF10     - RPCEYNSKIYQNGESFQPNCKHQCTCIDGAVGCIPLCPQELSLPNLGCPS -147
             :: .   .:  :::::  :: ::::.::::::.:::  .. ::   ::
βIG-M2    - APCVFGGSVYRSGESFQSSCKYQCTCLDGAVGCVPLCSMDVRLPSPDCPF -149

CEF10     - PRLVKVPGQCCEEWVCDESKDALEELEGFFSKEFGLDASEGELTRNNELI -197
            :: ::.:: ::::::::: ::                ..  :
βIG-M2    - PRRVKLPGKCCEEWVCDEPKDR---------------TAVGP-------- -176

CEF10     - AIVKGGLKMLPVFGSEPQSRAFENPKCIVQTTSWSQCSKTCGTGISTRVT -247
            :.    :     :: .:         :.:::: :: :::::: :::::::
βIG-M2    - ALAAYRLE--DTFGPDPTM---MRANCLVQTTEWSACSKTCGMGISTRVT -221

CEF10     - NDNPDCKLIKETRICEVRPCGQPSYASLKKGKKCTKTKKSPSPVRFTYAG -297
            :::  :.: : .:.: ::::       .:::::: .: :   ::.:  .:
βIG-M2    - NDNTFCRLEKQSRLCMVRPCEADLEENIKKGKKCIRTPKIAKPVKFELSG -271

CEF10     - CSSVKKYRPKYCGSCVDGRCCTPQQTRTVKIRFRCDDGETFTKSVMHIQS -347
            :.::: :: :.:: : :::::::  : :. . :.: :::   : .: : .
βIG-M2    - CTSVKTYRAKFCGVCTDGRCCTPHRTTTLPVEFKCPDGEIMKKNMMFIKT -321

CEF10     - CRCNYNCPHANEAYP--FYRLVNDIHKFRD -375
            : : ::::  :. .   .:: .
βIG-M2    - CACHYNCPGDNDIFESLYYRKMYG---DMA -348
```

FIGURE 5

```
βIG-M1    - MSSSTFRTLAVAVTLLHL-TRLALST-CPAACHCPLEA-PKCAPGVGLVR -47
            : .:     ...:. :: : :: :     : : : :  :: : :  :: ::
βIG-M2    - MLASVAGPISLALVLLALCTRPATGQDCSAQCQCAAEAAPHCPAGVSLVL -50

βIG-M1    - DGCGCCKVCAKQLNEDCSKTQPCDHTKGLECNFGASSTALKGICRAQSEG -97
            ::::::.:::::: : :.   :::  ::: : ::. .     :.: :  .:
βIG-M2    - DGCGCCRVCAKQLGELCTERDPCDPHKGLFCDFGSPANRKIGVCTAK-DG -99

βIG-M1    - RPCEYNSRIYQNGESFQPNCKHQCTCIDGAVGCIPLCPQELSLPNLGCPN -147
             :: .   .: :::::  :: ::::.:::::::.:::  .. ::    ::
βIG-M2    - APCVFGGSVYRSGESFQSSCKYQCTCLDGAVGCVPLCSMDVRLPSPDCPF -149

βIG-M1    - PRLVKVSGQCCEEWVCDEDSIKDSLDDQDDLLGLDASEVELTRNNELIAI -197
            :: ::. : :::::::::    . ..         : :  .: :
βIG-M2    - PRRVKLPGKCCEEWVCDEPKDRTAVG-----PALAAYRLEDT-------- -186

βIG-M1    - GKGSSLKRLPVFGTEPRVLFNPLHAHGQKCIVQTTSWSQCSKSCGTGIST -247
                        :: .:     . :   :.:::: :: :::.:: ::::
βIG-M2    - -----------FGPDP----TMMRAN---CLVQTTEWSACSKTCGMGIST -218

βIG-M1    - RVTNDNPECRLVKETRICEVRPCGQPVYSSLKKGKKCSKTKKSPEPVRFT -297
            ::::::  ::: : .:.: ::::    .   .:::::: .: :    ::.:
βIG-M2    - RVTNDNTFCRLEKQSRLCMVRPCEADLEENIKKGKKCIRTPKIAKPVKFE -268

βIG-M1    - YAGCSSVKKYRPKYCGSCVDGRCCTPLQTRTVKMRFRCEDGEMFSKNVMM -347
             .::.::: :: :.:: : :::::::  : :. . :.: :::.   ::.:
βIG-M2    - LSGCTSVKTYRAKFCGVCTDGRCCTPHRTTTLPVEFKCPDGEIMKKNMMF -318

βIG-M1    - IQSCKCNYNCPHPNEASFRLYSLFNDIHKFRD -379
            : .: : ::::  :.     ::  .  .
βIG-M2    - IKTCACHYNCPGDNDIFESLY--YRKMYGDMA -348
```

FIGURE 6

```
βIG-M1      CIVQTTSWSQCSKSCGTGISTRVT-------NDNPECRL-VKETRICEVR   42
CEF12CS     CIVQTTSWSQCSKTCGTGISTRVT-------NDNPDCKL-IKETRICEVR   42
βIG-M2      CLVQTTEWSACSKTCGMGISTRVT-------NDNTFCRL-EKQSRLCMVR   42
PFALCIPACS  NSI-STEWSPCSVTCGNGIQVRIKPGSANKPKDELDYEN-DIEKKICKME   48
PROPERDCSR  WSX-WSPWSPCSVTCSXGXQXXXRXRXCXXPAPXX-GXPCAGXAXXXXXQ   48
THROMBOCS   WSH-WSPWSSCSVTCGDGV--ITRIRLCNSPSPQMNGKPC--ECEARETK   45
PFALTRAPCS  CGV-WDEWSPCSVTCGKGTRSRKREILHEG---------CTSEIQEQ---   37
C7COMPCS    WDF-YAPWSECN-GCTKTQTRRRSVAVYG----QYGGQPCVG--NAFETQ   42
                 . ** *. .*. .
                  region II of CS protein
```

```
βIG-M1      PCGQPVYSSLKKGKKCSK   60
CEF12CS     PCGQPSYASLKKGKKCTK   60
βIG-M2      PCEADLEENIKKGKKCIR   60
PFALCIPACS  KCSSVF-----------N   55
PROPERDCSR  ACXXXXPCPXX-G-----   60
THROMBOCS   ACKKDA-CPIN-G-----   56
PFALTRAPCS  -CE-EERCPPKWE-----   48
C7COMPCS    SCEPTRGCPTEEG--C--   56
             *
```

FIGURE 7

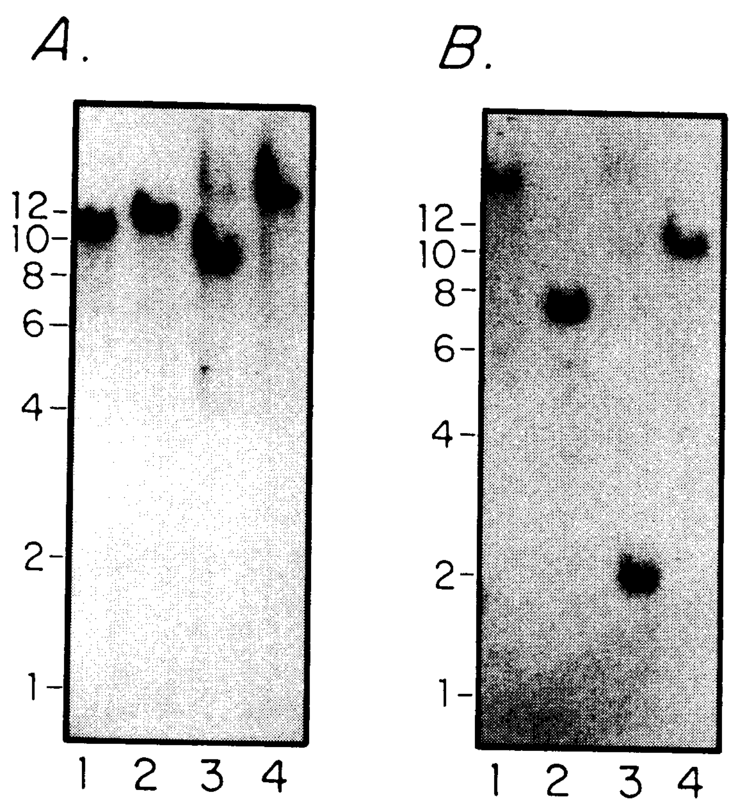


Figure 8

# SEQUENCE LISTING

(1) GENERAL INFORMATION:

(i) APPLICANT: BRISTOL-MYERS SQUIBB COMPANY
345 Park Avenue
New York, New York 10154
United States of America

(ii) TITLE OF INVENTION: TGF-BETA INDUCED GENE FAMILY

(iii) NUMBER OF SEQUENCES: 6

(iv) CORRESPONDENCE ADDRESS:
(A) ADDRESSEE: Joseph M. Sorrentino
(B) STREET: 3005 First Avenue
(C) CITY: Seattle
(D) STATE: Washington
(E) COUNTRY: USA
(F) ZIP: 98121

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.24

(vi) CURRENT APPLICATION DATA:
(A) APPLICATION NUMBER: US unassigned
(B) FILING DATE: 18-JAN-1991
(C) CLASSIFICATION:

(viii) ATTORNEY/AGENT INFORMATION:
(A) NAME: Sorrentino, Joseph M.
(B) REGISTRATION NUMBER: 32,598
(C) REFERENCE/DOCKET NUMBER: ON0081-

(ix) TELECOMMUNICATION INFORMATION:
(A) TELEPHONE: (206)728-4800
(B) TELEFAX: (206)448-4775

(2) INFORMATION FOR SEQ ID NO:1:

(i) SEQUENCE CHARACTERISTICS:
(A) LENGTH: 2028 base pairs
(B) TYPE: nucleic acid
(C) STRANDEDNESS: double
(D) TOPOLOGY: linear

(ii) MOLECULE TYPE: cDNA

(iii) HYPOTHETICAL: N

```
    (iv) ANTI-SENSE: N

    (vi) ORIGINAL SOURCE:
          (A) ORGANISM: Mus musculus
          (G) CELL TYPE: Fibroblast
          (H) CELL LINE: AKR2B

  (viii) POSITION IN GENOME:
          (C) UNITS: bp

    (ix) FEATURE:
          (A) NAME/KEY: CDS
          (B) LOCATION: 186..1322
          (D) OTHER INFORMATION:
    (ix) FEATURE:
          (A) NAME/KEY: mat_peptide
          (B) LOCATION: 186..1322
          (D) OTHER INFORMATION:


    (xi) SEQUENCE DESCRIPTION: SEQ ID NO:1:

GACCGTGAGC GAGAGGCCCA GAGAAGCGCC TGCAATCTCT GCGCTCCTCC GCCAGCACCT     60

CGAGAGAAGG ACACCCGCCG CCTCGGCCCT CGCCTCACCG CACTCCGGGC GCATTTGATC    120

CCGCTGCTCG CCGGCTTGTT GGTTCTGTGT CGCCGCGCTC GCCCCGGTTC CTCCTGCGCG    180

CCACA ATG AGC TCC AGC ACC TTC AGG ACG CTC GCT GTC GCC GTC ACC        227
      Met Ser Ser Ser Thr Phe Arg Thr Leu Ala Val Ala Val Thr
        1               5                  10

CTT CTC CAC TTG ACC AGA CTG GCG CTC TCC ACC TGC CCC GCC GCC TGC      275
Leu Leu His Leu Thr Arg Leu Ala Leu Ser Thr Cys Pro Ala Ala Cys
 15                  20                  25                  30

CAC TGC CCT CTG GAG GCA CCC AAG TGC GCC CCG GGA GTC GGG TTG GTC      323
His Cys Pro Leu Glu Ala Pro Lys Cys Ala Pro Gly Val Gly Leu Val
                 35                  40                  45

CGG GAC GGC TGC GGC TGC TGT AAG GTC TGC GCT AAA CAA CTC AAC GAG      371
Arg Asp Gly Cys Gly Cys Cys Lys Val Cys Ala Lys Gln Leu Asn Glu
             50                  55                  60

GAC TGC AGC AAA ACT CAG CCC TGC GAC CAC ACC AAG GGG TTG GAA TGC      419
Asp Cys Ser Lys Thr Gln Pro Cys Asp His Thr Lys Gly Leu Glu Cys
         65                  70                  75

AAT TTC GGC GCC AGC TCC ACC GCT CTG AAA GGG ATC TGC AGA GCT CAG      467
Asn Phe Gly Ala Ser Ser Thr Ala Leu Lys Gly Ile Cys Arg Ala Gln
     80                  85                  90

TCA GAA GGC AGA CCC TGT GAA TAT AAC TCC AGA ATC TAC CAA AAC GGG      515
Ser Glu Gly Arg Pro Cys Glu Tyr Asn Ser Arg Ile Tyr Gln Asn Gly
 95                 100                 105                 110
```

```
GAA AGC TTC CAG CCC AAC TGT AAA CAC CAG TGC ACA TGT ATT GAT GGC      563
Glu Ser Phe Gln Pro Asn Cys Lys His Gln Cys Thr Cys Ile Asp Gly
            115                 120                 125

GCC GTG GGC TGC ATT CCT CTG TGT CCC CAA GAA CTG TCT CTC CCC AAT      611
Ala Val Gly Cys Ile Pro Leu Cys Pro Gln Glu Leu Ser Leu Pro Asn
        130                 135                 140

CTG GGC TGT CCC AAC CCC CGG CTG GTG AAA GTC AGC GGG CAG TGC TGT      659
Leu Gly Cys Pro Asn Pro Arg Leu Val Lys Val Ser Gly Gln Cys Cys
    145                 150                 155

GAA GAG TGG GTT TGT GAT GAA GAC AGC ATT AAG GAC TCC CTG GAC GAC      707
Glu Glu Trp Val Cys Asp Glu Asp Ser Ile Lys Asp Ser Leu Asp Asp
    160                 165                 170

CAG GAT GAC CTC CTC GGA CTC GAT GCC TCG GAG GTG GAG TTA ACG AGA      755
Gln Asp Asp Leu Leu Gly Leu Asp Ala Ser Glu Val Glu Leu Thr Arg
175                 180                 185                 190

AAC AAT GAG TTA ATC GCA ATT GGA AAA GGC AGC TCA CTG AAG AGG CTT      803
Asn Asn Glu Leu Ile Ala Ile Gly Lys Gly Ser Ser Leu Lys Arg Leu
            195                 200                 205

CCT GTC TTT GGC ACC GAA CCG CGA GTT CTT TTC AAC CCT CTG CAC GCC      851
Pro Val Phe Gly Thr Glu Pro Arg Val Leu Phe Asn Pro Leu His Ala
        210                 215                 220

CAT GGC CAG AAA TGC ATC GTT CAG ACC ACG TCT TGG TCC CAG TGC TCC      899
His Gly Gln Lys Cys Ile Val Gln Thr Thr Ser Trp Ser Gln Cys Ser
    225                 230                 235

AAG AGC TGC GGA ACT GGC ATC TCC ACA CGA GTT ACC AAT GAC AAC CCA      947
Lys Ser Cys Gly Thr Gly Ile Ser Thr Arg Val Thr Asn Asp Asn Pro
    240                 245                 250

GAG TGC CGC CTG GTG AAA GAG ACC CGG ATC TGT GAA GTG CGT CCT TGT      995
Glu Cys Arg Leu Val Lys Glu Thr Arg Ile Cys Glu Val Arg Pro Cys
255                 260                 265                 270

GGA CAA CCA GTG TAC AGC AGC CTA AAA AAG GGC AAG AAA TGC AGC AAG     1043
Gly Gln Pro Val Tyr Ser Ser Leu Lys Lys Gly Lys Lys Cys Ser Lys
            275                 280                 285

ACC AAG AAA TCC CCA GAA CCA GTC AGA TTT ACT TAT GCA GGA TGC TCC     1091
Thr Lys Lys Ser Pro Glu Pro Val Arg Phe Thr Tyr Ala Gly Cys Ser
        290                 295                 300

AGT GTC AAG AAA TAC CGG CCC AAA TAC TGC GGC TCC TGC GTA GAT GGC     1139
Ser Val Lys Lys Tyr Arg Pro Lys Tyr Cys Gly Ser Cys Val Asp Gly
    305                 310                 315

CGG TGC TGC ACA CCT CTG CAG ACC AGA ACT GTG AAG ATG CGG TTC CGA     1187
Arg Cys Cys Thr Pro Leu Gln Thr Arg Thr Val Lys Met Arg Phe Arg
    320                 325                 330
```

```
TGC GAA GAT GGA GAG ATG TTT TCC AAG AAT GTC ATG ATG ATC CAG TCC        1235
Cys Glu Asp Gly Glu Met Phe Ser Lys Asn Val Met Met Ile Gln Ser
335                 340                 345                 350

TGC AAA TGT AAC TAC AAC TGC CCG CAT CCC AAC GAG GCA TCG TTC CGA        1283
Cys Lys Cys Asn Tyr Asn Cys Pro His Pro Asn Glu Ala Ser Phe Arg
                355                 360                 365

CTG TAC AGC CTA TTC AAT GAC ATC CAC AAG TTC AGG GAC TAAGTGCCTC         1332
Leu Tyr Ser Leu Phe Asn Asp Ile His Lys Phe Arg Asp
            370                 375

CAGGGTTCCT AGTGTGGGCT GGACAGAGGA GAAGCGCAAG CATCATGGAG ACGTGGGTGG      1392

GCGGAGGATG AATGGTGCCT TGCTCATTCT TGAGTAGCAT TAGGGTATTT CAAAACTGCC      1452

AAGGGGCTGA TGTGGACGGA CAGCAGCGCA GCCGCAGTTG GAGAATGCCA AGGGGCTGAT      1512

GTGGACGGAC AGCAGCGCAG CCGCAGTTGG AGAAGACTTC GCTTCATAGT ACTGGAGCGG      1572

GCATTATTGC TCCATATTGG AGCATGTTTA CGGATGACGT TCTGTTTTCT GTTTGTAAAT      1632

TATTTGCTAA GTGTATTTTT TTGCTCCAGA CCCCCCCCCC CCCTTTCTTG GTTCTACAAT      1692

TGTAATAGAG ACAAAATAAG ATTAGTTGGG CCAAGTGAAA GCCCTGCTTG TCCTTTGACA      1752

GAAGTAAATG AAAGCGCCTC TCATTCCTTC CCGAGCGGAG GGGGGACACT CTGTGAGTGT      1812

CCTTGGGGCA GCTACCTGCA CTCTAAAACT GCAAACAGAA ACCAGGTGTT TTAAGATTGA      1872

ATGTTTTTTT ATTTATCAAA GTGTAGCTTT TGGGGAGGGA GGGGAAATGT AATACTGGAA      1932

TAATTTGTAA ATGATTTTAA TTTTATATCA GTGAAGAGAA TTTATTTATA AAATTAATCA      1992

TTTAATAAAG AAATATTTAC CTAAAAAAAA AAAAAA                                2028
```

(2) INFORMATION FOR SEQ ID NO:2:

(i) SEQUENCE CHARACTERISTICS:
(A) LENGTH: 379 amino acids
(B) TYPE: amino acid
(D) TOPOLOGY: linear

(ii) MOLECULE TYPE: protein

(xi) SEQUENCE DESCRIPTION: SEQ ID NO:2:

```
Met Ser Ser Ser Thr Phe Arg Thr Leu Ala Val Ala Val Thr Leu Leu
  1               5                  10                  15

His Leu Thr Arg Leu Ala Leu Ser Thr Cys Pro Ala Ala Cys His Cys
            20                  25                  30

Pro Leu Glu Ala Pro Lys Cys Ala Pro Gly Val Gly Leu Val Arg Asp
        35                  40                  45
```

```
Gly Cys Gly Cys Cys Lys Val Cys Ala Lys Gln Leu Asn Glu Asp Cys
     50                  55                  60

Ser Lys Thr Gln Pro Cys Asp His Thr Lys Gly Leu Glu Cys Asn Phe
 65                  70                  75                  80

Gly Ala Ser Ser Thr Ala Leu Lys Gly Ile Cys Arg Ala Gln Ser Glu
                 85                  90                  95

Gly Arg Pro Cys Glu Tyr Asn Ser Arg Ile Tyr Gln Asn Gly Glu Ser
            100                 105                 110

Phe Gln Pro Asn Cys Lys His Gln Cys Thr Cys Ile Asp Gly Ala Val
        115                 120                 125

Gly Cys Ile Pro Leu Cys Pro Gln Glu Leu Ser Leu Pro Asn Leu Gly
    130                 135                 140

Cys Pro Asn Pro Arg Leu Val Lys Val Ser Gly Gln Cys Cys Glu Glu
145                 150                 155                 160

Trp Val Cys Asp Glu Asp Ser Ile Lys Asp Ser Leu Asp Asp Gln Asp
                165                 170                 175

Asp Leu Leu Gly Leu Asp Ala Ser Glu Val Glu Leu Thr Arg Asn Asn
            180                 185                 190

Glu Leu Ile Ala Ile Gly Lys Gly Ser Ser Leu Lys Arg Leu Pro Val
        195                 200                 205

Phe Gly Thr Glu Pro Arg Val Leu Phe Asn Pro Leu His Ala His Gly
    210                 215                 220

Gln Lys Cys Ile Val Gln Thr Thr Ser Trp Ser Gln Cys Ser Lys Ser
225                 230                 235                 240

Cys Gly Thr Gly Ile Ser Thr Arg Val Thr Asn Asp Asn Pro Glu Cys
                245                 250                 255

Arg Leu Val Lys Glu Thr Arg Ile Cys Glu Val Arg Pro Cys Gly Gln
            260                 265                 270

Pro Val Tyr Ser Ser Leu Lys Lys Gly Lys Lys Cys Ser Lys Thr Lys
        275                 280                 285

Lys Ser Pro Glu Pro Val Arg Phe Thr Tyr Ala Gly Cys Ser Ser Val
    290                 295                 300

Lys Lys Tyr Arg Pro Lys Tyr Cys Gly Ser Cys Val Asp Gly Arg Cys
305                 310                 315                 320

Cys Thr Pro Leu Gln Thr Arg Thr Val Lys Met Arg Phe Arg Cys Glu
                325                 330                 335
```

```
Asp Gly Glu Met Phe Ser Lys Asn Val Met Met Ile Gln Ser Cys Lys
            340                 345                 350

Cys Asn Tyr Asn Cys Pro His Pro Asn Glu Ala Ser Phe Arg Leu Tyr
        355                 360                 365

Ser Leu Phe Asn Asp Ile His Lys Phe Arg Asp
    370                 375
```

(2) INFORMATION FOR SEQ ID NO:3:

(i) SEQUENCE CHARACTERISTICS:
(A) LENGTH: 2330 base pairs
(B) TYPE: nucleic acid
(C) STRANDEDNESS: double
(D) TOPOLOGY: linear

(ii) MOLECULE TYPE: cDNA

(iii) HYPOTHETICAL: N

(iv) ANTI-SENSE: N

(vi) ORIGINAL SOURCE:
(A) ORGANISM: Mus musculus
(G) CELL TYPE: Fibroblast
(H) CELL LINE: AKR2B

(viii) POSITION IN GENOME:
(C) UNITS: bp

(ix) FEATURE:
(A) NAME/KEY: CDS
(B) LOCATION: 204..1247
(D) OTHER INFORMATION:

(ix) FEATURE:
(A) NAME/KEY: mat_peptide
(B) LOCATION: 204..1247
(D) OTHER INFORMATION:

(xi) SEQUENCE DESCRIPTION: SEQ ID NO:3:

```
AGACTCAGCC AGATCCACTC CAGCTCCGAC CCCAGGAGAC CGACCTCCTC CAGACGGCAG      60

CAGCCCCAGC CCAGCCGACA ACCCCAGACG CCACCGCCTG GAGCGTCCAG ACACCAACCT     120

CCGCCCCTGT CCGAATCCAG GCTCCAGCCG CGCCTCTCGT CGCCTCTGCA CCCTGCTGTG     180

CATCCTCCTA CCGCGTCCCG ATC ATG CTC GCC TCC GTC GCA GGT CCC ATC         230
                          Met Leu Ala Ser Val Ala Gly Pro Ile
                           1               5
```

```
AGC CTC GCC TTG GTG CTC CTC GCC CTC TGC ACC CGG CCT GCT ACG GGC     278
Ser Leu Ala Leu Val Leu Leu Ala Leu Cys Thr Arg Pro Ala Thr Gly
 10                  15                  20                  25

CAG GAC TGC AGC GCG CAA TGT CAG TGC GCA GCC GAA GCA GCG CCG CAC     326
Gln Asp Cys Ser Ala Gln Cys Gln Cys Ala Ala Glu Ala Ala Pro His
                 30                  35                  40

TGC CCC GCC GGC GTG AGC CTG GTG CTG GAC GGC TGC GGC TGC TGC CGC     374
Cys Pro Ala Gly Val Ser Leu Val Leu Asp Gly Cys Gly Cys Cys Arg
             45                  50                  55

GTC TGC GCC AAG CAG CTG GGA GAA CTG TGT ACG GAG CGT GAC CCC TGC     422
Val Cys Ala Lys Gln Leu Gly Glu Leu Cys Thr Glu Arg Asp Pro Cys
         60                  65                  70

GAC CCA CAC AAG GGC CTC TTC TGC GAT TTC GGC TCC CCC GCC AAC CGC     470
Asp Pro His Lys Gly Leu Phe Cys Asp Phe Gly Ser Pro Ala Asn Arg
     75                  80                  85

AAG ATT GGA GTG TGC ACT GCC AAA GAT GGT GCA CCC TGT GTC TTC GGT     518
Lys Ile Gly Val Cys Thr Ala Lys Asp Gly Ala Pro Cys Val Phe Gly
 90                  95                 100                 105

GGG TCG GTG TAC CGC AGC GGT GAG TCC TTC CAA AGC AGC TGC AAA TAC     566
Gly Ser Val Tyr Arg Ser Gly Glu Ser Phe Gln Ser Ser Cys Lys Tyr
                110                 115                 120

CAA TGC ACT TGC CTG GAT GGG GCC GTG GGC TGC GTG CCC CTA TGC AGC     614
Gln Cys Thr Cys Leu Asp Gly Ala Val Gly Cys Val Pro Leu Cys Ser
            125                 130                 135

ATG GAC GTG CGC CTG CCC AGC CCT GAC TGC CCC TTC CCG AGA AGG GTC     662
Met Asp Val Arg Leu Pro Ser Pro Asp Cys Pro Phe Pro Arg Arg Val
        140                 145                 150

AAG CTG CCT GGG AAA TGC TGC GAG GAG TGG GTG TGT GAC GAG CCC AAG     710
Lys Leu Pro Gly Lys Cys Cys Glu Glu Trp Val Cys Asp Glu Pro Lys
    155                 160                 165

GAC CGC ACA GCA GTT GGC CCT GCC CTA GCT GCC TAC CGA CTG GAA GAC     758
Asp Arg Thr Ala Val Gly Pro Ala Leu Ala Ala Tyr Arg Leu Glu Asp
170                 175                 180                 185

ACA TTT GGC CCA GAC CCA ACT ATG ATG CGA GCC AAC TGC CTG GTC CAG     806
Thr Phe Gly Pro Asp Pro Thr Met Met Arg Ala Asn Cys Leu Val Gln
                190                 195                 200

ACC ACA GAG TGG AGC GCC TGT TCT AAG ACC TGT GGA ATG GGC ATC TCC     854
Thr Thr Glu Trp Ser Ala Cys Ser Lys Thr Cys Gly Met Gly Ile Ser
            205                 210                 215

ACC CGA GTT ACC AAT GAC AAT ACC TTC TGC AGA CTG GAG AAG CAG AGC     902
Thr Arg Val Thr Asn Asp Asn Thr Phe Cys Arg Leu Glu Lys Gln Ser
        220                 225                 230
```

```
CGC CTC TGC ATG GTC AGG CCC TGC GAA GCT GAC CTG GAG GAA AAC ATT         950
Arg Leu Cys Met Val Arg Pro Cys Glu Ala Asp Leu Glu Glu Asn Ile
    235                 240                 245

AAG AAG GGC AAA AAG TGC ATC CGG ACA CCT AAA ATC GCC AAG CCT GTC         998
Lys Lys Gly Lys Lys Cys Ile Arg Thr Pro Lys Ile Ala Lys Pro Val
250                 255                 260                 265

AAG TTT GAG CTT TCT GGC TGC ACC AGT GTG AAG ACA TAC AGG GCT AAG        1046
Lys Phe Glu Leu Ser Gly Cys Thr Ser Val Lys Thr Tyr Arg Ala Lys
                270                 275                 280

TTC TGC GGG GTG TGC ACA GAC GGC CGC TGC TGC ACA CCG CAC AGA ACC        1094
Phe Cys Gly Val Cys Thr Asp Gly Arg Cys Cys Thr Pro His Arg Thr
            285                 290                 295

ACC ACT CTG CCA GTG GAG TTC AAA TGC CCC GAT GGC GAG ATC ATG AAA        1142
Thr Thr Leu Pro Val Glu Phe Lys Cys Pro Asp Gly Glu Ile Met Lys
        300                 305                 310

AAG AAT ATG ATG TTC ATC AAG ACC TGT GCC TGC CAT TAC AAC TGT CCT        1190
Lys Asn Met Met Phe Ile Lys Thr Cys Ala Cys His Tyr Asn Cys Pro
    315                 320                 325

GGG GAC AAT GAC ATC TTT GAG TCC CTG TAC TAC AGG AAG ATG TAC GGA        1238
Gly Asp Asn Asp Ile Phe Glu Ser Leu Tyr Tyr Arg Lys Met Tyr Gly
330                 335                 340                 345

GAC ATG GCG TAAAGCCAGG AAGTAAGGGA CACGAACTCA TTAGACTATA                1287
Asp Met Ala

ACTTGAACTG AGTTGCATCT CATTTTCTTC TGTAAAAACA ATTACAGTAG CACATTAATT      1347

TAAATCTGTG TTTTTAACTA CCGTGGGAGG AACTATCCCA CCAAAGTGAG AACGTTATGT      1407

CATGGCCATA CAAGTAGTCT GTCAACCTCA GACACTGGTT TCGAGACAGT TTACACTTGA      1467

CAGTTGTTCA TTAGCGCACA GTGCCAGAAC GCACACTGAG GTGAGTCTCC TGGAACAGTG      1527

GAGATGCCAG GAGAAAGAAA GACAGGTACT AGCTGAGGTT ATTTTAAAAG CAGCAGTGTG      1587

CCTACTTTTT GGAGTGTAAC CGGGGAGGGA AATTATAGCA TGCTTGCAGA CAGACCTGCT      1647

CTAGCGAGAG CTGAGCATGT GTCCTCCACT AGATGAGGCT GAGTCCAGCT GTTCTTTAAG      1707

AACAGCAGTT TCAGCTCTGA CCATTCTGAT TCCAGTGACA CTTGTCAGGA GTCAGAGCCT      1767

TGTCTGTTAG ACTGGACAGC TTGTGGCAAG TAAGTTTGCC TGTAACAAGC CAGATTTTTA      1827

TTGATATTGT AAATATTGTG GATATATATA TATATATATA TATATTTGTA CAGTTATCTA      1887

AGTTAATTTA AAGTCATTTG TTTTTGTTTT AAGTGCTTTT GGGATTTTAA ACTGATAGCC      1947

TCAAACTCCA AACACCATAG GTAGGACACG AAGCTTATCT GTGATTCAAA ACAAAGGAGA      2007
```

```
TACTGCAGTG GGAATTGTGA CCTGAGTGAC TCTCTGTCAG AACAAACAAA TGCTGTGCAG    2067

GTGATAAAGC TATGTATTGG AAGTCAGATT TCTAGTAGGA AATGTGGTCA AATCCCTGTT    2127

GGTGAACAAA TGGCCTTTAT TAAGAAATGG CTGGCTCAGG GTAAGGTCCG ATTCCTACCA    2187

GGAAGTGCTT GCTGCTTCTT TGATTATGAC TGGTTTGGGG TGGGGGGCAG TTTATTTGTT    2247

GAGAGTGTGA CCAAAAGTTA CATGTTTGCA CTTTCTAGTT GAAAATAAAG TATATATATA    2307

TTTTTATATG AAAAAAAAAA AAA                                            2330
```

(2) INFORMATION FOR SEQ ID NO:4:

(i) SEQUENCE CHARACTERISTICS:
(A) LENGTH: 348 amino acids
(B) TYPE: amino acid
(D) TOPOLOGY: linear

(ii) MOLECULE TYPE: protein

(xi) SEQUENCE DESCRIPTION: SEQ ID NO:4:

```
Met Leu Ala Ser Val Ala Gly Pro Ile Ser Leu Ala Leu Val Leu Leu
 1               5                  10                  15

Ala Leu Cys Thr Arg Pro Ala Thr Gly Gln Asp Cys Ser Ala Gln Cys
            20                  25                  30

Gln Cys Ala Ala Glu Ala Ala Pro His Cys Pro Ala Gly Val Ser Leu
        35                  40                  45

Val Leu Asp Gly Cys Gly Cys Cys Arg Val Cys Ala Lys Gln Leu Gly
    50                  55                  60

Glu Leu Cys Thr Glu Arg Asp Pro Cys Asp Pro His Lys Gly Leu Phe
65                  70                  75                  80

Cys Asp Phe Gly Ser Pro Ala Asn Arg Lys Ile Gly Val Cys Thr Ala
                85                  90                  95

Lys Asp Gly Ala Pro Cys Val Phe Gly Gly Ser Val Tyr Arg Ser Gly
            100                 105                 110

Glu Ser Phe Gln Ser Ser Cys Lys Tyr Gln Cys Thr Cys Leu Asp Gly
        115                 120                 125

Ala Val Gly Cys Val Pro Leu Cys Ser Met Asp Val Arg Leu Pro Ser
    130                 135                 140

Pro Asp Cys Pro Phe Pro Arg Arg Val Lys Leu Pro Gly Lys Cys Cys
145                 150                 155                 160

Glu Glu Trp Val Cys Asp Glu Pro Lys Asp Arg Thr Ala Val Gly Pro
                165                 170                 175
```

Ala Leu Ala Ala Tyr Arg Leu Glu Asp Thr Phe Gly Pro Asp Pro Thr
180 185 190

Met Met Arg Ala Asn Cys Leu Val Gln Thr Thr Glu Trp Ser Ala Cys
195 200 205

Ser Lys Thr Cys Gly Met Gly Ile Ser Thr Arg Val Thr Asn Asp Asn
210 215 220

Thr Phe Cys Arg Leu Glu Lys Gln Ser Arg Leu Cys Met Val Arg Pro
225 230 235 240

Cys Glu Ala Asp Leu Glu Glu Asn Ile Lys Lys Gly Lys Lys Cys Ile
245 250 255

Arg Thr Pro Lys Ile Ala Lys Pro Val Lys Phe Glu Leu Ser Gly Cys
260 265 270

Thr Ser Val Lys Thr Tyr Arg Ala Lys Phe Cys Gly Val Cys Thr Asp
275 280 285

Gly Arg Cys Cys Thr Pro His Arg Thr Thr Thr Leu Pro Val Glu Phe
290 295 300

Lys Cys Pro Asp Gly Glu Ile Met Lys Lys Asn Met Met Phe Ile Lys
305 310 315 320

Thr Cys Ala Cys His Tyr Asn Cys Pro Gly Asp Asn Asp Ile Phe Glu
325 330 335

Ser Leu Tyr Tyr Arg Lys Met Tyr Gly Asp Met Ala
340 345

(2) INFORMATION FOR SEQ ID NO:5:

(i) SEQUENCE CHARACTERISTICS:
(A) LENGTH: 1804 base pairs
(B) TYPE: nucleic acid
(C) STRANDEDNESS: double
(D) TOPOLOGY: linear

(ii) MOLECULE TYPE: cDNA

(iii) HYPOTHETICAL: N

(iv) ANTI-SENSE: N

(vi) ORIGINAL SOURCE:
(A) ORGANISM: Gallus domesticus
(G) CELL TYPE: Fibroblast
(H) CELL LINE: CEF10

(viii) POSITION IN GENOME:
(C) UNITS: bp

(ix) FEATURE:
(A) NAME/KEY: CDS
(B) LOCATION: 53..1177
(D) OTHER INFORMATION:
(ix) FEATURE:
(A) NAME/KEY: mat_peptide
(B) LOCATION: 119..1177
(D) OTHER INFORMATION:
(ix) FEATURE:
(A) NAME/KEY: sig_peptide
(B) LOCATION: 53..118
(D) OTHER INFORMATION:

(x) PUBLICATION INFORMATION:
(A) AUTHORS: Simmons , Daniel L
Levy, Daniel B
Yannoni, Yvonne
Erikson, R L
(B) TITLE: Identification of a phorbal ester-repressible
v-src-inducible gene
(C) JOURNAL: Proc. Natl. Acad. Sci. U.S.A.
(D) VOLUME: 86
(F) PAGES: 1178-1182
(G) DATE: February-1989
(K) RELEVANT RESIDUES IN SEQ ID NO:5: FROM 1 TO 1804

(xi) SEQUENCE DESCRIPTION: SEQ ID NO:5:

```
CCCGCTTCGC GATCGCGTCT CGAGCTCCGC TCTCGCTCCG CGCCGCTAAG AC ATG        55
                                                          Met
                                                          -22

GGC TCT GCG GGA GCT CGC CCC GCG CTG GCG GCC GCC CTG CTC TGC CTG     103
Gly Ser Ala Gly Ala Arg Pro Ala Leu Ala Ala Ala Leu Leu Cys Leu
    -20                 -15                 -10

GCC CGC CTG GCT CTC GGC TCT CCG TGC CCC GCC GTC TGC CAG TGC CCG     151
Ala Arg Leu Ala Leu Gly Ser Pro Cys Pro Ala Val Cys Gln Cys Pro
 -5                   1               5                  10

GCC GCC GCG CCG CAG TGC GCC CCG GGC GTG GGG CTG GTG CCG GAC GGC     199
Ala Ala Ala Pro Gln Cys Ala Pro Gly Val Gly Leu Val Pro Asp Gly
            15                  20                  25

TGC GGC TGC TGC AAG GTC TGC GCC AAG CAG CTG AAC GAG GAC TGC AGC     247
Cys Gly Cys Cys Lys Val Cys Ala Lys Gln Leu Asn Glu Asp Cys Ser
        30                  35                  40

CGG ACG CAG CCC TGC GAC CAC ACC AAG GGG CTG GAG TGC AAC TTC GGC     295
Arg Thr Gln Pro Cys Asp His Thr Lys Gly Leu Glu Cys Asn Phe Gly
    45                  50                  55
```

GCC AGC CCC GCC GCC ACC AAC GGC ATC TGC AGA GCA CAG TCT GAG GGG 343
Ala Ser Pro Ala Ala Thr Asn Gly Ile Cys Arg Ala Gln Ser Glu Gly
60 65 70 75

AGA CCA TGC GAA TAC AAC TCC AAA ATC TAC CAG AAC GGC GAA AGC TTC 391
Arg Pro Cys Glu Tyr Asn Ser Lys Ile Tyr Gln Asn Gly Glu Ser Phe
80 85 90

CAG CCC AAC TGC AAG CAC CAG TGT ACG TGC ATA GAT GGA GCT GTG GGC 439
Gln Pro Asn Cys Lys His Gln Cys Thr Cys Ile Asp Gly Ala Val Gly
95 100 105

TGC ATC CCG CTC TGC CCG CAG GAG CTC TCC CTC CCC AAC CTG GGC TGC 487
Cys Ile Pro Leu Cys Pro Gln Glu Leu Ser Leu Pro Asn Leu Gly Cys
110 115 120

CCC AGC CCC AGG CTG GTC AAA GTG CCT GGG CAG TGC TGC GAG GAG TGG 535
Pro Ser Pro Arg Leu Val Lys Val Pro Gly Gln Cys Cys Glu Glu Trp
125 130 135

GTC TGC GAT GAG AGC AAG GAT GCG CTG GAG GAG CTG GAG GGC TTC TTC 583
Val Cys Asp Glu Ser Lys Asp Ala Leu Glu Glu Leu Glu Gly Phe Phe
140 145 150 155

AGC AAG GAG TTT GGT CTG GAC GCT TCT GAG GGC GAA CTG ACC CGG AAC 631
Ser Lys Glu Phe Gly Leu Asp Ala Ser Glu Gly Glu Leu Thr Arg Asn
160 165 170

AAC GAG CTG ATT GCC ATC GTG AAG GGA GGC CTG AAG ATG CTA CCT GTT 679
Asn Glu Leu Ile Ala Ile Val Lys Gly Gly Leu Lys Met Leu Pro Val
175 180 185

TTT GGA TCC GAG CCG CAA AGC CGA GCT TTT GAG AAT CCC AAA TGC ATT 727
Phe Gly Ser Glu Pro Gln Ser Arg Ala Phe Glu Asn Pro Lys Cys Ile
190 195 200

GTG CAA ACA ACT TCC TGG TCC CAG TGC TCA AAG ACG TGT GGG ACC GGC 775
Val Gln Thr Thr Ser Trp Ser Gln Cys Ser Lys Thr Cys Gly Thr Gly
205 210 215

ATC TCC ACC AGA GTC ACC AAC GAC AAT CCC GAC TGC AAG CTC ATC AAA 823
Ile Ser Thr Arg Val Thr Asn Asp Asn Pro Asp Cys Lys Leu Ile Lys
220 225 230 235

GAG ACC AGG ATA TGC GAA GTG AGG CCG TGT GGC CAG CCC AGC TAC GCC 871
Glu Thr Arg Ile Cys Glu Val Arg Pro Cys Gly Gln Pro Ser Tyr Ala
240 245 250

TCC CTG AAG AAG GGA AAA AAA TGT ACC AAG ACT AAG AAG TCC CCA TCC 919
Ser Leu Lys Lys Gly Lys Lys Cys Thr Lys Thr Lys Lys Ser Pro Ser
255 260 265

CCT GTA AGG TTT ACT TAT GCT GGA TGC TCC AGT GTG AAG AAG TAC CGC 967
Pro Val Arg Phe Thr Tyr Ala Gly Cys Ser Ser Val Lys Lys Tyr Arg
270 275 280

```
CCC AAG TAC TGT GGG TCT TGC GTG GAT GGC AGG TGC TGT ACT CCC CAG      1015
Pro Lys Tyr Cys Gly Ser Cys Val Asp Gly Arg Cys Cys Thr Pro Gln
    285                 290                 295

CAG ACC AGG ACT GTC AAG ATC CGT TTC CGC TGC GAT GAT GGA GAA ACC      1063
Gln Thr Arg Thr Val Lys Ile Arg Phe Arg Cys Asp Asp Gly Glu Thr
300                 305                 310                 315

TTC ACC AAG AGT GTC ATG ATG ATC CAG TCC TGC CGC TGC AAC TAC AAC      1111
Phe Thr Lys Ser Val Met Met Ile Gln Ser Cys Arg Cys Asn Tyr Asn
                320                 325                 330

TGT CCG CAT GCA AAC GAA GCT TAT CCC TTC TAC AGA CTG GTC AAT GAC      1159
Cys Pro His Ala Asn Glu Ala Tyr Pro Phe Tyr Arg Leu Val Asn Asp
            335                 340                 345

ATC CAC AAA TTT AGG GAC TAAGTGGTAT TTGGGGTGGG ATGTTAAACA             1207
Ile His Lys Phe Arg Asp
        350

GAATTCTGAA GTAACCAGCC ATGGAGAAAG GACCTCTGAT GGAAGTGGTG CCTTGCCCCA    1267

TTTGAGGGCA ATATGAGATA TTACAGGAGT GCACTGTGCA ACTGGACACT AATGCGACAG    1327

AGATTTAAGC ATACTTAAAG CTTCATAGTA CTGGAGCAAC CTTACTGCTT CTTTTTGGAG    1387

CACCTTTATC TTACACTGTT TTCTGTTTGT AAGTGATCTG ATGTTTTGTT CCGGTTATGA    1447

AAGCTCTTCC TCTCCCGTTC AGTTTAACAC TACGCTTTTC CCCTCCCCTC CATCTTCTCC    1507

CCTACTCTCC CAACCAAGTT GGAAGTTACA TTCCTTCCTG AGGTGGGCAC TTGTGGGGTG    1567

TTCACAGTGG CAGCTATTAT GTACCAACTG TAGTTTAATG GCAAACAGAA ATCAGTTGTT    1627

TTAAAGCTGA GTATTTTATT TATCAAACTG TAGCTCTTTT GTTTTCTTTT TTTTTTTTTT    1687

TAACCCCTTC CAACCCCTGT AATACTGGAA TAAGTTGTAA ATGATTTTAA TTTTATATTC    1747

GATGAATTAA AAGAATTTAT TTATGGAATT AATCATTTAA TAAAGAAATA TTTACCT       1804
```

(2) INFORMATION FOR SEQ ID NO:6:

(i) SEQUENCE CHARACTERISTICS:
(A) LENGTH: 375 amino acids
(B) TYPE: amino acid
(D) TOPOLOGY: linear

(ii) MOLECULE TYPE: protein

(xi) SEQUENCE DESCRIPTION: SEQ ID NO:6:

```
Met Gly Ser Ala Gly Ala Arg Pro Ala Leu Ala Ala Ala Leu Leu Cys
-22     -20                 -15                 -10
```

```
Leu Ala Arg Leu Ala Leu Gly Ser Pro Cys Pro Ala Val Cys Gln Cys
     -5                   1               5                  10

Pro Ala Ala Ala Pro Gln Cys Ala Pro Gly Val Gly Leu Val Pro Asp
                 15                  20                  25

Gly Cys Gly Cys Cys Lys Val Cys Ala Lys Gln Leu Asn Glu Asp Cys
             30                  35                  40

Ser Arg Thr Gln Pro Cys Asp His Thr Lys Gly Leu Glu Cys Asn Phe
         45                  50                  55

Gly Ala Ser Pro Ala Ala Thr Asn Gly Ile Cys Arg Ala Gln Ser Glu
     60                  65                  70

Gly Arg Pro Cys Glu Tyr Asn Ser Lys Ile Tyr Gln Asn Gly Glu Ser
 75                  80                  85                  90

Phe Gln Pro Asn Cys Lys His Gln Cys Thr Cys Ile Asp Gly Ala Val
                 95                 100                 105

Gly Cys Ile Pro Leu Cys Pro Gln Glu Leu Ser Leu Pro Asn Leu Gly
            110                 115                 120

Cys Pro Ser Pro Arg Leu Val Lys Val Pro Gly Gln Cys Cys Glu Glu
        125                 130                 135

Trp Val Cys Asp Glu Ser Lys Asp Ala Leu Glu Glu Leu Glu Gly Phe
    140                 145                 150

Phe Ser Lys Glu Phe Gly Leu Asp Ala Ser Glu Gly Glu Leu Thr Arg
155                 160                 165                 170

Asn Asn Glu Leu Ile Ala Ile Val Lys Gly Gly Leu Lys Met Leu Pro
                175                 180                 185

Val Phe Gly Ser Glu Pro Gln Ser Arg Ala Phe Glu Asn Pro Lys Cys
            190                 195                 200

Ile Val Gln Thr Thr Ser Trp Ser Gln Cys Ser Lys Thr Cys Gly Thr
        205                 210                 215

Gly Ile Ser Thr Arg Val Thr Asn Asp Asn Pro Asp Cys Lys Leu Ile
    220                 225                 230

Lys Glu Thr Arg Ile Cys Glu Val Arg Pro Cys Gly Gln Pro Ser Tyr
235                 240                 245                 250

Ala Ser Leu Lys Lys Gly Lys Lys Cys Thr Lys Thr Lys Lys Ser Pro
                255                 260                 265

Ser Pro Val Arg Phe Thr Tyr Ala Gly Cys Ser Ser Val Lys Lys Tyr
            270                 275                 280
```

```
Arg Pro Lys Tyr Cys Gly Ser Cys Val Asp Gly Arg Cys Cys Thr Pro
        285                 290                 295

Gln Gln Thr Arg Thr Val Lys Ile Arg Phe Arg Cys Asp Asp Gly Glu
    300                 305                 310

Thr Phe Thr Lys Ser Val Met Met Ile Gln Ser Cys Arg Cys Asn Tyr
315                 320                 325                 330

Asn Cys Pro His Ala Asn Glu Ala Tyr Pro Phe Tyr Arg Leu Val Asn
                335                 340                 345

Asp Ile His Lys Phe Arg Asp
            350
```